(19) Europäisches Patentamt
European Patent Office
Office européen des brevets

(11) EP 0 520 281 B1

(12) **EUROPÄISCHE PATENTSCHRIFT**

(45) Veröffentlichungstag und Bekanntmachung des Hinweises auf die Patenterteilung:
**12.03.1997 Patentblatt 1997/11**

(51) Int Cl.[6]: **G02F 1/37**, C07C 211/44

(21) Anmeldenummer: **92110062.4**

(22) Anmeldetag: **15.06.1992**

(54) **Optisches Element zur effizienten Erzeugung kurzwelligen Laserlichtes**

Optical element for generating short wave laser light

Dispositif optique pour la génération de lumière laser de courte longueur d'onde

(84) Benannte Vertragsstaaten:
**CH DE FR GB IT LI NL**

(30) Priorität: **25.06.1991 CH 1878/91**
**10.01.1992 CH 65/92**

(43) Veröffentlichungstag der Anmeldung:
**30.12.1992 Patentblatt 1992/53**

(73) Patentinhaber: **F. HOFFMANN-LA ROCHE AG**
**4002 Basel (CH)**

(72) Erfinder:
- **Buchecker, Richard**
  **CH-8008 Zürich (CH)**
- **Chen, Xin-Hua**
  **W-1000 Berlin (DE)**
- **Fünfschilling, Jürg**
  **CH-4054 Basle (CH)**
- **Herr, Rolf-Peter**
  **W-7800 Freiburg (DE)**
- **Schadt, Martin**
  **CH-4411 Seltisberg (CH)**
- **Schmitt, Klaus**
  **W-7850 Lörrach (DE)**

(74) Vertreter: **Buntz, Gerhard et al**
**F.Hoffmann-La Roche AG**
**Patent Department (PLP),**
**124 Grenzacherstrasse**
**4070 Basel (CH)**

(56) Entgegenhaltungen:
**EP-A- 0 301 511** **EP-A- 0 304 738**
**EP-A- 0 338 845** **US-A- 4 865 406**

- **NONLINEAR OPTICS: DIGEST, Kauai, Hawaii, 16. - 20. Juli 1990, Seiten 147-148; M.G. Robinson et al.: "Second harmonic generation in guided ferroelelectric liquid cristal structure"**
- **OPTICS LETTERS, Band 15, Nr. 5, März 1990, Seiten 267-269, New York, US; J.Y. LIU et al.: "Second harmonic generation in ferroelectric..."**
- **JAPANESE JOURNAL OF APPLIED PHYSICS, Band 28, Nr. 6, Juni 1989, Seiten L997- L999, Tokyo, JP; A. TAGUCHI et al.: "Angle phase matching in ..."**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein optisches Element zur effizienten Erzeugung kurzwelligen Laserlichtes durch Frequenzkonversion mit einer wellenleitenden Schicht aus einem flüssigkristallinen Material, das eine periodische Struktur aufweist, die eine Quasi-Phasenanpassung eines geführten Laserstrahls erlaubt, wobei die Periodenlänge der räumlichen Struktur durch die Kohärenzlänge $l_c = \pi/\Delta\beta$ des Materials definiert ist, worin $\Delta\beta = \beta_0 (2\omega)-2 \beta_0 (\omega)$ ist, mit $\omega$ = Kreisfrequenz der Fundamentalwelle, 0 = Mode nullter Ordnung und $\beta$ = Ausbreitungskonstante der Mode. Die Erfindung betrifft ausserdem zur Herstellung dieser Schicht geeignete Verbindungen.

In einer Vielzahl von Anwendungsgebieten geht derzeit die Entwicklung dahin, elektronische oder opto-mechanische Bauelemente durch elektrooptische oder rein optische auf nichtlinear-optischen ("nlo") Effekten beruhende Bauelemente zu ersetzen, weil diese im Prinzip schneller, zuverlässiger und wirtschaftlicher arbeiten als jene. Viele dieser Anwendungen, z.B. optische Speichersysteme mit hoher Datendichte würden kompakte, robuste Laserlichtquellen mit Emission im Bereich von < 500 nm erfordern. Da kurzwellige Laserdioden nicht verfügbar und bisher nicht realisierbar sind, wird seit langem versucht, mit Hilfe nichtlinear-optischer Methoden, insbesondere Frequenzverdopplung ("Second Harmonic Generation: SHG"), Summenfrequenzbildung etc., langwelliges Licht herkömmlicher Laserdioden effizient in kurzwelliges umzuwandeln. Dabei wären Techniken, die die Integration der Laserdiode mit dem nlo-aktiven Material erlauben, besonders attraktiv.

Eine effiziente Frequenzumwandlung kann nur erreicht werden, wenn es gelingt, im nlo-aktiven Medium die sog. Phasenanpassungs ("Phasematching") -Bedingung für das eingestrahlte langwellige Licht und das erzeugte kurzwellige Licht zu erfüllen. Dies kann mit geeigneten nichtlinearen doppelbrechenden Kristallen, wie z.B. KDP, ADP, $LiNbO_3$, etc. erreicht werden.

Da die Konversioneffizienz quadratisch von der Leistungsdichte der Grundwelle abhängt, sind bei den relativ geringen Lichtintensitäten der verfügbaren Laserdioden unpraktikabel grosse und kostspielige nlo-Kristalle erforderlich. Viel günstiger sind die Verhältnisse, wenn für die Umwandlung anstelle solcher Kristalle Wellenleiterkonfigurationen verwendet werden können. In diesen wird das Licht auf einen kleinen Querschnitt komprimiert und damit die Leistungsdichte um Grössenordnungen erhöht. Darüberhinaus erleichtert die Wellenleitungsgeometrie, die Phasematching-Bedingung zu realisieren. Drei mögliche Phasematching-Techniken seien erwähnt:

1. Ausnutzung der Dispersionseigenschaft verschiedener Moden im Wellenleiter. Bei richtiger Dimensionierung des Wellenleiters wird die Phasematching-Bedingung für die in niedriger Modenordnung geführte Grundwelle und die in einer höheren Mode geführte 2. Harmonischen erfüllt. Allerdings hängt die Konversionseffizienz quadratisch von der Grösse des Ueberlappungsintegrals der zwei Moden ab und ist bei Moden verschiedener Ordnung klein.

2. Ausnutzung der Doppelbrechungseigenschaft anisotroper Wellenleiter. Diese Methode entspricht der Situation in doppelbrechenden Kristallen und ist nur auf Materialien mit geeigneter Doppelbrechung anwendbar. In diesem Fall ist Konversion zwischen Moden gleicher Ordnung (z.B. TE $\rightarrow$ TM) möglich und damit sind grosse Werte des Ueberlappungsintegrals realisierbar.

3. Ausnutzung periodischer Strukturen. Diese unter dem Namen Quasi-Phasematching bekannte Methode moduliert die Nichtlinearität des Wellenleitermaterials räumlich periodisch. Diese Methode hat in neuerer Zeit an Interesse gewonnen, da sie sich relativ einfach in gepolten Strukturen (z.B. gepolte nlo-Polymere) realisieren lässt. Auch hier findet die Konversion zwischen Moden gleicher Ordnung (z.B. $TE_o^{(\omega)} \rightarrow TE_o^{(2\omega)}$) statt.

Für die erwähnten Wellenleiter potentiell geeignete $\chi^{(2)}$-aktive Materialen sind nach dem aktuellen Stand der Technik im wesentlichen die folgenden Substanzklassen:

1. Einkristalline anorganische Schichten.
   Ein typisches Beispiel ist das $LiNbO_3$, das u.a. wegen seiner grossen nichtlinearen Koeffizienten $d_{31}$ = - 5.95 pm/V und $d_{33} \equiv$ - 37 pm/V besonders intensiv untersucht worden ist und häufig als Referenzmaterial für die Beurteilung von nlo-Substanzen herangezogen wird. Nachteilig ist jedoch der Umstand, dass solche Schichten schlecht zu präparieren und sehr teuer sind.

2. $\chi^{(2)}$-aktive Langmuir-Blodgett-Multischichten. Diese haben aber den Nachteil, dass sie instabil, vor allem nicht temperaturstabil, sind.

3. Gepolte $\chi^{(2)}$-aktive Polymere. Sie haben aber den Nachteil, dass sie nicht langzeitstabil sind.

In US-A-4,865,406 sind solche frequenzverdoppelnden optischen Wellenleiter beschrieben, die aus dünnen

Schichten gepolter flüssigkristalliner Polymere bestehen. Die wellenleitenden Schichten aus nlo-Material besitzen eine periodische Struktur, die eine Quasi-Phasenanpassung eines geführten Laserstrahles erlaubt. Derartige gepolte Schichten besitzen eine starke Tendenz zur Relaxation.

Aus EP-A-338 845 ist ein nlo-Material bekannt, das sich für die Frequenzverdoppelung eignet. Es handelt sich dabei um ein FLC-Polymer mit sehr kleiner $\chi^{(2)}$-Aktivität, das mit bekannten nlo-Chromophoren dotiert ist. In einem derartigen Guest-Host-System wird nicht die für eine technisch nützliche Anwendung notwendige nlo-Effizienz erreicht.

Der Erfindung liegt die Aufgabe zugrunde, ein frequenzkonvertierendes optisches Element zur Verfügung zu stellen, dessen wellenleitende Schicht die vorstehenden Nachteile nicht aufweist.

Erfindungsgemäss wurde dies erreicht durch ein optisches Element der eingangs genannten Art, in welchem die Periodenlänge der räumlichen Struktur dem Doppelten der Kohärenzlänge $l_c$ entspricht und dass das flüssigkristalline Material entweder eine ferroelektrische, $\chi^{(2)}$-aktive Mischung chiraler Moleküle mit einer nlo-Molekülgruppe, deren $\chi^{(2)}$-aktive Achse im wesentlichen quer zur Längsachse des chiralen Moleküls gerichtet ist, und $S_c$-Phasen bildenden Flüssigkristallmolekülen, oder ein $\chi^{(2)}$-aktives, $S_c$*-Phasen bildendes Seitenkettencopolymer, welches chirale Seitenketten mit einer nlo-Molekülgruppe besitzt, deren $\chi^{(2)}$-aktive Achse im wesentlichen quer zur Längsachse der Seitenkette gerichtet ist, enthält.

Gemäss einer alternativen Ausführungsform der Erfindung erlaubt das Material aufgrund seiner Doppelbrechungseigenschaft Phasenanpassung der $d_{21}$ - Komponente des nlo-Tensors.

Gemäss einer weiteren Ausführungsform bildet das ferroelektrische Flüssigkristallmaterial eine $S_C$*-Phase oder eine höher geordnete ferroelektrische Phase, die im elektrischen Feld dipolar orientiert werden kann.

Gemäss einer weiteren Ausführungsform bildet das ferroelektrische Flüssigkristallmaterial eine $S_C$*-Phase, die durch Randkräfte bistabil homogen orientiert wird. Dies geschieht in der SSF-Konfiguration mit Materialien, die eine grossen Helixganghöhe aufweisen oder mit SBF-Materialien.

Vorzugsweise besteht das ferroelektrische Flüssigkristallmaterial aus einer Mischung chiraler nlo-aktiver Moleküle, die $S_C$*-Phasen bilden.

Vorzugsweise ist die dipolare Orientierung durch Abkühlen unter die Glastemperatur fixiert.

Gemäss einer weiteren Ausführungsform arbeitet das optische Element durch Ausnutzung des Pockelseffektes als elektrooptischer Schalter oder Lichtmodulator im Spektralbereich von 1300 bis 430 nm.

Vorzugsweise weist die Mischung die Phasenfolge I-Chol-$Sm_A$-$S_C$*-Glas auf. Das Element kann in der $S_C$*-Phase betrieben werden, derart, dass bei Anlegen eines elektrischen Feldes die $\chi^{(2)}$-Eigenschaft wirksam wird und bei Ausschalten des elektrischen Feldes verschwindet.

Das optische Element kann speziell zur Frequenzverdopplung von Laserlicht der Wellenlänge 850-1300 nm, vorzugsweise 900 - 1300 nm, dienen und zur Bildung eines frequenzverdoppelnden Moduls mit einer Laserdiode mit Emission im Bereich von 850-1300 nm optisch gekoppelt sein.

Gemäss einer weiteren Ausführungsform steht die dipolar ausgerichtete Achse senkrecht auf der Wellenleiterebene

Ferroelektrische Flüssigkristalle weisen für die Nutzung nichtlinearer Effekte 2.Ordnung die folgenden sehr attraktiven Eigenschaften auf. Sie verbinden die Eigenschaft relativ einfacher Präparation und Eignung zum Aufbau der für Bauelemente erforderlichen Strukturen, welche die gepolten nlo-Polymere auszeichnet, mit der für $\chi^{(2)}$-aktive Einkristalle charakteristischen Eigenschaft der nichtzentrosymmetrischen Struktur.

Im Prinzip sind sowohl niedermolekulare als auch polymere ferroelektrische Flüssigkristalle Kandidaten für $\chi^{(2)}$-aktive Materialien, da sie in der ferroelektrischen Phase, z.B der chiralen smektisch C Phase ($S_C$*) eine nichtzentrosymmetrische Struktur annehmen. Unter geeigneten Bedingungen bilden diese Substanzen in der $S_C$*-Phase eine Helixstruktur aus, die z.B. durch Anlegen eines kleinen elektrischen Feldes in eine ebene dipolar orientierte Struktur überführt werden kann. Dabei stehen die ausgerichteten Dipole senkrecht zum Direktor. Der Ordnungsgrad einer solchen Struktur kann grosse Werte annehmen.

Diese Eigenschaften ferroelektrischer Flüssigkristalle sind gut untersucht und bilden die Grundlage für die Anwendung in Flüssigkristallanzeigen (LCD). Als Beispiele seien auf dem sog. Surface Stabilized Ferroelectric-Effekt (SSF) [vgl. Clark N.A. et al., Appl.Phys.Lett. 36 (1980), 899] und auf dem sog. Short-Pitch Bistable Ferrroelectric-Effekt (SBF) [vgl. Fünfschilling J. et al., Jpn.J.Appl.Phys. 30 (1991) 741] beruhende Flüssigkristallanzeigen genannt.

Dünne $\chi^{(2)}$-aktive Filme aus niedermolekularen ferroelektrischen Flüssigkristallmischungen haben bisher nur geringe Beachtung gefunden. Dies beruht darauf, dass alle bisher publizierten SHG-Untersuchungen an ferroelektrischen Flüssigkristall-Mischungen so niedrige nichtlineare Koeffizienten (z.B. $d_{22}$ = 0.027 pm/V; $d_{23}$ = 0.07pm/V; $d_{16}$ = 0.0026 pm/V Ref. 4) zeigen, dass ihre technische Verwendung als SHG-Material nicht sinnvoll erschien.

Ferroelektrische Flüssigkristallmischungen bestehen entweder aus chiralen mesogenen Molekülen, die $S_C$*-Phasen ausbilden, oder aus einer Kombination von $S_c$-Phasen bildenden mesogenen Molekülen mit chiralen Dotierstoffen, die geeignet sind, die $S_C$*-Phase zu induzieren.

Effiziente $\chi^{(2)}$-aktive ferroelektrische Flüssigkristall-Materialien können nun dadurch erzeugt werden, dass in die oben genannten chiralen Substanzen nlo-aktive Strukturelemente eingebaut werden, die eine hohe Hyperpolarisierbarkeit 2. Ordnung aufweisen, derart dass die für die Nichtlinearität relevante Achse in der ferroelektrischen Flüssigkristallkonfiguration dipolar ausgerichtet wird.

Es ist bekannt, dass Molekülgruppen der Form A Π D, wobei A einen Elektronenakzeptor, Π eine Brücke mit delokalisiertem Elektronensystem und D einen Elektronendonator bedeuten, entlang der Achse Donator-Akzeptor hyperpolarisierbar sind. Ein Beispiel für ein solches Molekül ist das Paranitroanilin.

Da mesogene Moleküle, die ferroelektrische Flüssigkristallphasen bilden, stäbchenförmige Gestalt haben (Längenausdehnung >> Breite), muss die quer zum Direktor eingebaute nlo-Molekülgruppe kompakt sein, da sie anderenfalls die flüssigkristalline Phase zerstören würde. Dasselbe gilt für die chiralen nlo-Dotierstoffe. Diese Forderung beschränkt die erreichbare Hyperpolarisierbarkeit.

Ferner besteht ein direkter Zusammenhang zwischen der Grösse der Nichtlinearität und der kurzwelligen Grenze des transparenten Spektralbereichs des nlo-Moleküls. Um Frequenzkonversion in den blauen Spektralbereich hinein zu ermöglichen, ist Transparenz des Materials in diesem zu fordern.

Als nlo-aktives Strukturelement hat sich beispielsweise 5-Amino-2-nitro-1,4-phenylen als hervorragend geeignet erwiesen. Ueberraschend wurde nämlich gefunden, dass der Einbau dieses nlo-Strukturelements in ein geeignetes smektogenes Molekül zur Ausbildung der $S_C$*-Phase führt.

Es konnte ferner gezeigt werden, dass sich die für die Nichtlinearität der nlo-Gruppe relevante Achse in der $S_C$*-Phase hochgradig dipolar orientiert, falls die nlo-Einheit möglichst nahe bei der chiralen Gruppe des Flüssigkristallmoleküls oder des chiralen Dotierstoffmoleküls eingebaut wird.

SHG-Messungen (1064 nm-532 nm Konversion) ergeben für die nichtlinearen Koeffizienten $d_{22}$ und $d_{21}$ Werte in der Grössenordnung 10 pm/V bzw. 5 pm/V. Diese Werte sind $10^3$ mal grösser als die höchsten bisher publizierten Daten und sind mit denen von $LiNbO_3$ durchaus vergleichbar. Diesen grossen nichtlinearen Koeffizienten entsprechen die beobachteten hohen Werte der spontanen Polarisation (bis 700 nC/cm$^2$). Die Analyse der Messungen beweist eine nahezu perfekte dipolare Orientierung der Moleküle in der Schicht. Es wurde eine Klasse von Molekülen mit den oben beschriebenen Eigenschaften gefunden, deren Bauprinzip im folgenden beschrieben wird.

Es handelt sich hierbei um p-Nitro-Anilin Derivate der allgemeinen Formel

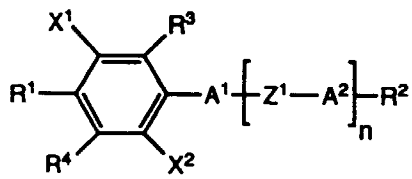

I

Hierin bedeuten:

$X^1$, $X^2$ : eines $-NO_2$ und das andere $-NR^5R^6$, worin $R^5$ und $R^6$ unabhängig voneinander H oder Niederalkyl darstellen;

$R^3$, $R^4$ : unabhängig voneinander H, F, Cl, Br, $NH_2$, $NO_2$, CN, Niederalkyl oder Niederalkoxy, mit der Massgabe, dass $X^1$ verschieden von $R^4$ und $X^2$ verschieden von $R^3$ ist;

$R^1$: chirales Alkyl oder Cycloalkyl worin, unabhängig voneinander, eine oder zwei nicht benachbarte $-CH_2$-Gruppen durch O, S, -COO-, -OOC-, -HC=CH-, und im aliphatischen Rest auch durch -C≡C- ersetzt sein können, und welches einfach oder mehrfach substituiert sein kann mit F, Cl, CN, Niederalkyl und Niederalkoxy, mit der Massgabe, dass sich das Chiralitätszentrum in einer Alkylkette am ersten oder zweiten C-Atom befindet und bei einem Cycloalkylrest an einem Ring-C-Atom befindet;

$A^1$: 1 bis 4 sechsgliedrige Ringe, welche miteinander bzw. mit dem p-Nitro-Anilinring direkt verknüpft sind und/oder an einer oder gegebenenfalls mehreren Stellen über $-CH_2$-$CH_2$-, $-CH_2O$-, $-OCH_2$-, -COO-, -OOC-, -COS-, -SOC-, -CH=CH-, -C≡C-, -N=N-, -N=NO-, -ON=N-, -CH=N-, -N=CH-, oder Butylen, worin eine $-CH_2CH_2$-Gruppe durch -COO-, -OOC-, -HC=CH-, -C≡C- und/oder eine $-CH_2$-Gruppe durch O oder S ersetzt sein kann, verknüpft sind. Die sechsgliedrigen Ringe in $A^1$ bedeuten unabhängig voneinander unsubstituiertes oder gegebenenfalls mit F, Cl, Niederalkyl oder Niederalkoxy mono-, di-, oder poly-substituiertes 1,4-Phenylen, in welchem 1 oder 2 -CH-Gruppen durch Stickstoff ersetzt sein können, oder 1,4-Cyclohexylen, in welchem eine oder zwei $-CH_2$- Gruppen (vorzugsweise nicht benachbarte) durch O oder S oder eine $-CH_2CH_2$- Gruppe durch -CH=CH- ersetzt sein können, oder Naphthalin-2,5-diyl, Decalin-2,6-diyl, Tetralin-2,6-diyl, Thiadiazolyl oder Oxodiazolyl;

Z1: eine einfache Kovalenzbindung, $-CH_2-CH_2-$, $-CH_2O-$, $-OCH_2-$, -COO-, -OOC-, -COS-, -SOC-, -CH=CH-, -C≡C-, -N=N-, -N=NO-, -ON=N-, -CH=N-, -N=CH- oder Butylen, worin eine $-CH_2CH_2$-Gruppe durch -COO-, -OOC-, -HC=CH-, -C≡C- und/oder eine $-CH_2$-Gruppe durch O oder S ersetzt sein können;

$A^2$: das Strukturelement

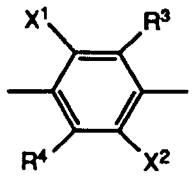

II

worin $X^1$, $X^2$, $R^3$ und $R^4$ die vorher beschriebene Bedeutung haben;

n: die Zahl 0 oder 1;

$R^2$: chirales oder achirales Alkyl oder Cycloalkyl worin, unabhängig voneinander, eine oder mehrere $-CH_2$-Gruppen durch O, S, -COO-, -HC=CH-, und im aliphatischen Rest auch durch -C≡C- oder 1,4-Phenylen ersetzt sein können, und welche einfach oder mehrfach substituiert sein können mit F, Cl, CN, Niederalkyl und Niederalkoxy, mit der Massgabe, dass sich ein allfällig vorhandenes Chiralitätszentrum in einer Alkylkette am ersten oder zweiten C-Atom befindet und bei einem Cycloalkylrest an einem Ring-C-Atom befindet, oder falls n die Zahl 0 bedeutet, auch die Gruppe

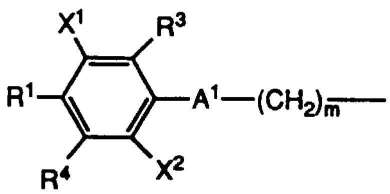

III

worin $X^1$, $X^2$, $R^1$, $R^3$, $R^4$ und $A^1$ obige Bedeutung haben und m eine ganze Zahl 6-16 bedeutet, worin eine oder zwei nicht benachbarte $-CH_2$-Gruppen unabhängig voneinander durch O, S, -COO-, -CH=CH- oder-C≡C- ersetzt sein können.

Bevorzugt werden Verbindungen der Formel I, worin $R^2$ chirales oder achirales Alkyl oder Cycloalkyl worin, unabhängig voneinander, eine oder zwei nicht benachbarte $-CH_2$-Gruppen durch -O-, -S-, -COO-, -HC=CH-, und im aliphatischen Rest auch -C≡C- ersetzt sein können, und welches einfach oder mehrfach substituiert sein kann mit F, Cl, CN, Niederalkyl und Niederalkoxy, mir der Massgabe, dass sich ein allfällig vorhandenes Chiralitätszentrum in einer Alkylkette am ersten oder zweiten C-Atom befindet und in einem Cycloalkylrest an einem Ring-C-Atom befindet, oder falls n die Zahl 0 bedeutet, auch die Gruppe

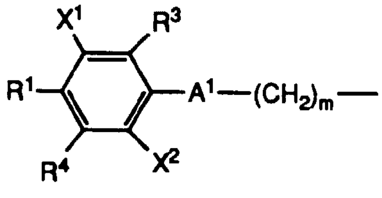

III

worin $X^1$, $X^2$, $R^1$, $R^3$, $R^4$ und $A^1$ die obige Bedeutung haben und m eine ganze Zahl von 6 bis 16 bedeutet, worin eine oder zwei nicht benachbarte $-CH_2$-Gruppen unabhängig voneinander durch -O-, -S-, -COO-, -HC=CH- oder -C≡C- ersetzt sein.

In den Verbindungen der obigen Formel I können, gewünschtenfalls, die Reste $R^1$ und $R^4$ und/oder - falls n=1 - auch $R^2$ und $R^3$ bzw. $R^2$ und $R^4$ vertauscht sein.

Der Ausdruck "chirales oder achirales Alkyl oder Cycloalkyl, worin, unabhängig voneinander, eine oder mehrere $-CH_2$-Gruppen durch O, S, COO-, -HC=CH-, und im aliphatischen Rest auch durch -C≡C- oder 1,4 Phenylen ersetzt sein können, und welches einfach oder mehrfach substituiert sein kann mit F, Cl, CN, Niederalkyl und Niederalkoxy" umfasst im Rahmen dieser Anmeldung geradkettige oder verzweigte Reste mit 1 bis 16, vorzugsweise 4 bis 12 Kohlenstoffatomen wie Alkyl, Alkoxy, Alkoxyalkyl, Alkenyl, Alkenyloxy, Alkenyloxyalkyl, Alkoxycarbonyl, Alkoxycarbonylalkoxy, Alkanoyloxy, 1-Haloalkyl, 2-Haloalkyl, 2-Haloalkyl, 2-Haloalkoxy, 2-Haloalkoxycarbonyl, 1-Cyanoalkyl, 2-Cyanoalkyl, 2-Cyanoalkyl, 2-Cyanoalkoxy, 2-Cyanoalkoxycarbonyl, 1-Methylalkyl, 2-Methylalkyl, 2-Methylalkyl, 2-Methylalkoxy, 2-Methylalkoxy-carbonyl, 1-Methoxyalkyl, 2-Methoxyalkyl, 2-Methoxyalkyl, 2-Methoxyalkoxy, 2-Methoxyalkoxycarbonyl, 8-Acryloyloxyoctanoxy, 9-Acryloyloxynonanoxy, 10-Acryloyloxydecanoxy, 11-Acryloyloxyundecanoxy, 12-Acryloyloxydodecanoxy, 8-Methacryloyloxyoctanoxy, 9-Methacryloyloxynonanoxy, 10-Methacryloyloxydecanoxy, 11-Methacryloyloxyundecanoxy, 12-Methacryloyloxydodecanoxy, 8-(2-Chloracryloyloxy)octanoxy, 9-(2-Chloracryloyloxy)nonanoxy, 10-(2-Chloracryloyloxy)decanoxy, 11-(2-Chloracryloyloxy)undecanoxy, 12-(2-Chloracryloyloxy)dodecanoxy, 8-Vinyloxyoctanoxy, 9-Vinyloxynonanoxy, 10-Vinyloxydecanoxy, 11-Vinyloxyundecanoxy, 12-Vinyloxydodecanoxy, 8-vinyloxycarbonyloctanoxy, 9-vinyloxycarbonylnonanoxy, 10-vinyloxycarbonyldecanoxy, 11-vinyloxycarbonyldecanoxy, 12-vinyloxycarbonyldodecanoxy, 8-(4-Styryl)oxyoctanoxy, 9-(4-Styryl)oxynonanoxy, 10-(4-Styryl)oxydecanoxy, 11-(4-Styryl)oxydecanoxy, 12-(4-Styryl)oxydodecanoxy, 8-(4-Styryl)methylenoxyoctanoxy, 9-(4-Styryl)methylenoxynonanoxy, 10(4-Styryl)methylenoxydecanoxy, 11-(4-Styryl)methylenodecanoxy, 12-(4-Styryl)methylenoxydodecanoxy, 7-Octenyloxy, 8-Nonenyloxy, 9-Decenyloxy, 10-Undecenyloxy, 11-Decenenyloxy und dergleichen, sowie 4 bis 7-gliedrige, gesättigte, gegebenenfalls chirale Ringe wie Cydopentyl, Cyclohexyl, 2-Oxiranyl, 2-Tetrahydropyran, 2-Tetrahydropyranyl, 2-Methylcyclohexyl, 2-Methylcyclopentyl und dergleichen.

Die Ausdrücke "Niederalkyl" bzw. "Niederalkoxy" bezeichnen im Rahmen der vorliegenden Erfindung geradkettige Alkyl- bzw. Alkoxygruppen mit 1 bis 4 Kohlenstoffatomen wie Methyl, Aethyl, Propyl, Butyl, Methoxy, Aethoxy, Propyloxy oder Butyloxy insbesondere Methyl, Aethyl bzw. Methoxy oder Aethoxy.

Der Ausdruck "unsubstituiertes oder mit F, Cl, Niederalkyl und/oder Niederalkoxy substituiertes 1,4-Phenylen, in welchem gegebenenfalls 1 oder 2 CH-Gruppen durch Stickstoff ersetzt sind", umfasst Gruppen wie 1,4-Phenylen, 2-Fluor-1,4-phenylen, 2,3-Difluor-1,4-phenylen, 2-Chlor-1,4-phenylen, 2-Methyl-1,4-phenylen, Pyridin-2,5-diyl, Pyrazin-2,5-diyl, Pyrimidin-2,5-diyl, Pyridazin-3,6-diyl und dergleichen. Bevorzugte Gruppen sind 1,4-Phenylen, 2-Fluor-1,4-phenylen, 2,3-Difluor-1,4-phenylen, Pyridin-2,5-diyl, Pyrazin-2,5-diyl, Pyrimidin-2,5-diyl und dergleichen.

Der Ausdruck "1,4-Cyclohexylen, in welchem eine oder zwei nicht benachbarte $-CH_2$-Gruppen durch O oder S oder eine $-CH_2CH_2$-Gruppe durch -CH=CH- ersetzt sein können" umfasst u.a trans-1,4-Cyclohexylen, 1,3-Dioxan-2,5-diyl, 1,3-Dithian-2,5-diyl und dergleichen.

Der Ausdruck "Tetralin-2,6-diyl" bezeichnet 1,2,3,4-Tetrahydro-naphthalin-2,6-diyl. Der Ausdruck "trans-Decalin-2,6-diyl" umfasst von trans-Decahydronaphthalin abgeleitete 2,6-disubstituierte Gruppen, insbesondere (4a$\alpha$H, 8a$\beta$H)-Decahydronaphthalin-2$\alpha$,6$\beta$-diyl, und dergleichen.

Die erfindungsgemässen nicht linear optischen Verbindungen tragen alle eine mehrfach substituierte p-Nitroanilinogruppe, welche durch Kombination mit bekannten, flüssigkristallinen Bausteinen die weiter oben beschriebenen herausragenden Eigenschaften dieser Verbindungsklasseen bewirken. p-Nitro-Aniline mit geeigneten Substitutionsmuster sind z.T. im Handel erhältlich oder können in an sich bekannter Weise hergestellt werden. In den nachfolgenden Syntheseschemata 1 und 2 und in den Beispielen 1 bis 5 werden zudem solche Herstellungsmethoden beschrieben.

## Schema I

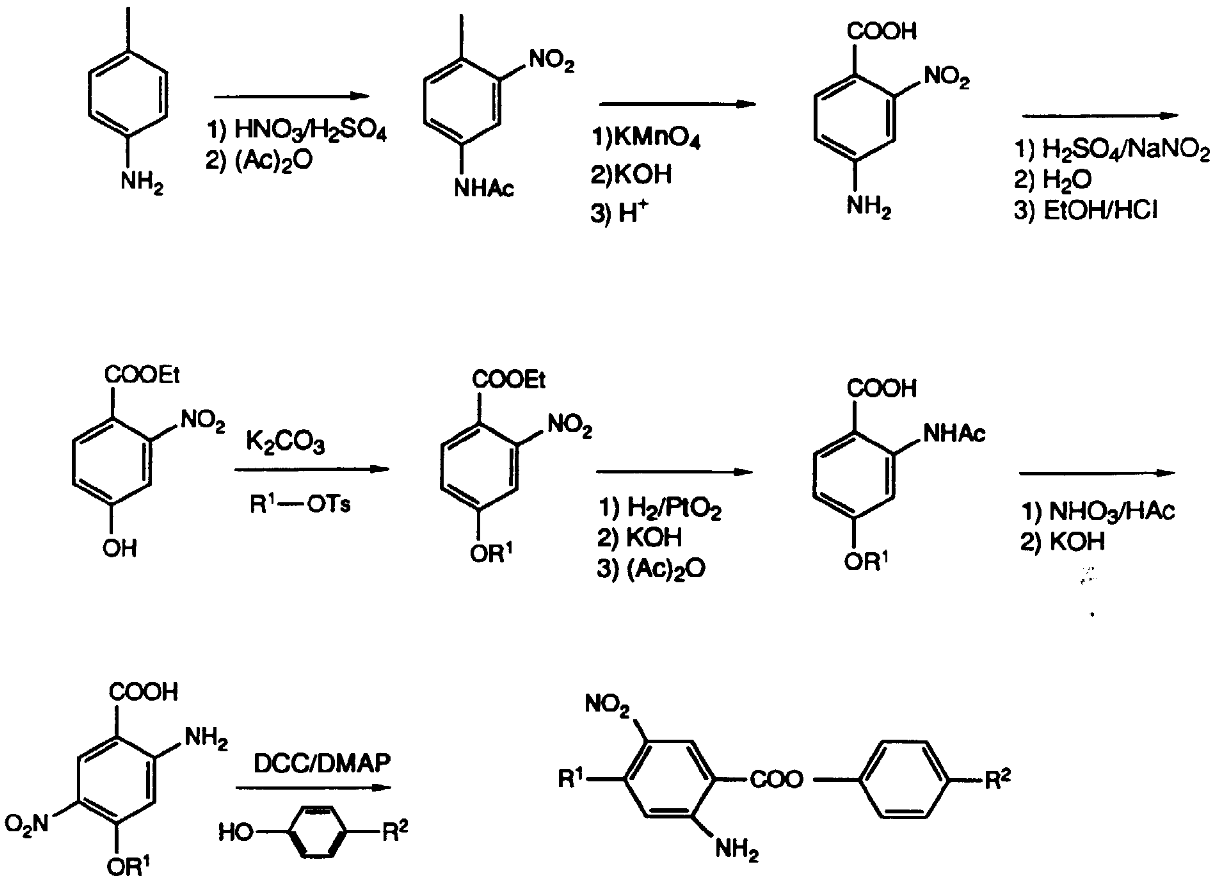


DCC = N,N'-Dicyclocarbodiimid
DMAP = 4-(Dimethylamino)pyridin

Schema II

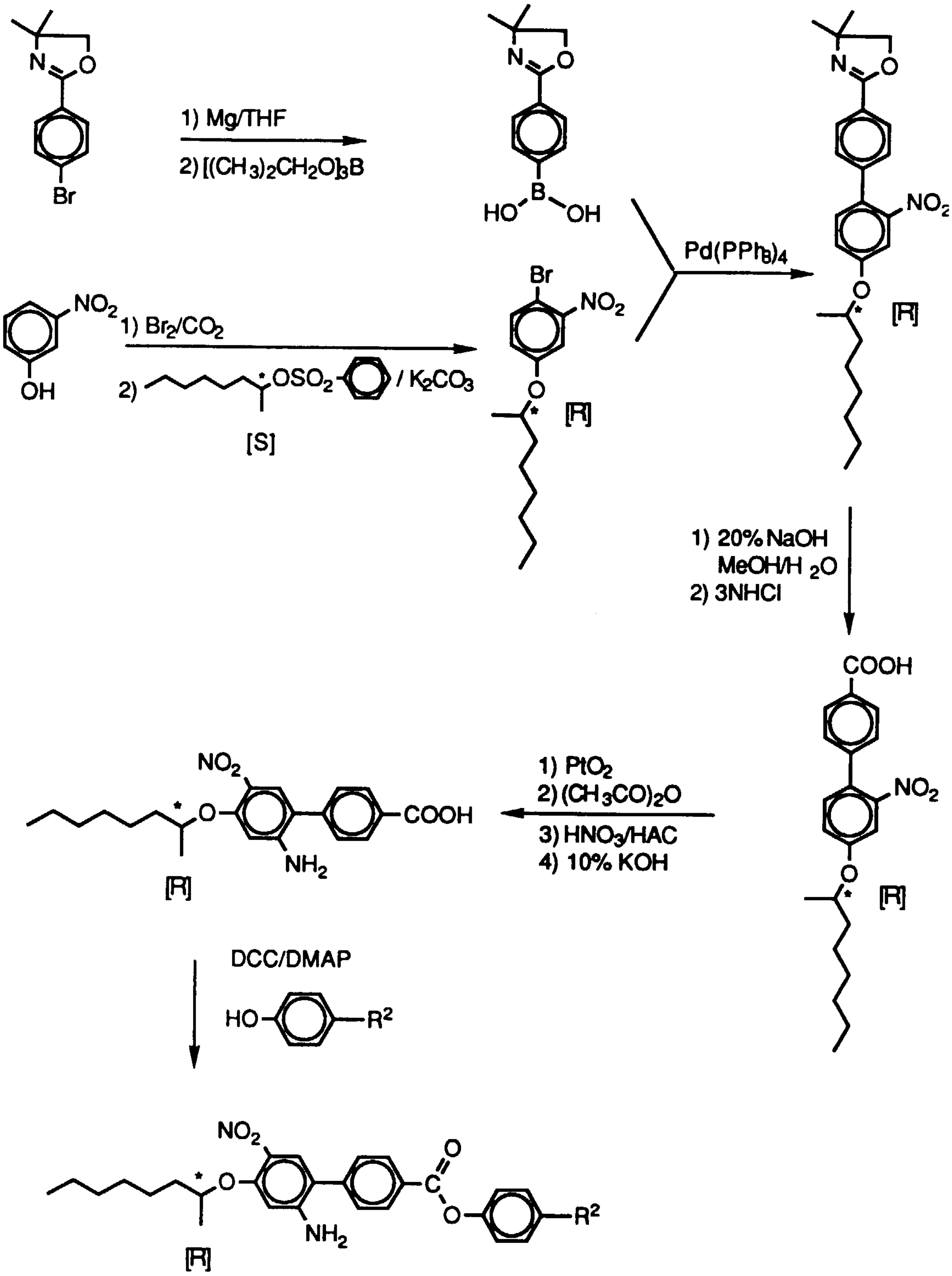


In diesen Schemata haben die einzelenen Substituenten die weiter vorne angegebene Bedeutung

Unter diesen sind Moleküle, die selbst $S_C$*-Phasen bilden und daher als solche als $\chi^{(2)}$-aktives Wellenleitermaterial verwendet werden können.

Eine zweite Gruppe bilden Moleküle, die zwar nicht selbst $S_C$*-Phasen ausbilden, aber als chiraler Dotierstoff $S_C$*-Phasen in geeigneten $S_C$-Matrixmischungen induzieren.

Ferner können Verbindungen der Formel I auch als Monomere zur Bildung von LC-Seitenketten Polymeren, welche eine ferroelektrische Phase aufweisen, verwendet werden. Zu diesem Zweck werden vorzugsweise Verbindungen der

allgemeinen Formel eingesetzt:

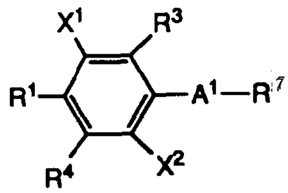

Ia

worin $X^1$, $X^2$, $R^3$, $R^4$, $R^1$ und $A^1$ die obengenannten Bedeutungen haben und $R^7$ chirales oder achirales Alkyl bedeutet, in welchem, unabhängig voneinander, eine oder mehrere $-CH_2$-Gruppen durch -O-, -S-, -COO-, -HC=CH-, -C≡C- oder 1,4-Phenylen ersetzt sein können, und welches einfach oder mehrfach substituiert sein kann mit F, Cl, CN, Niederalkyl und Niederalkoxy.

Besonders bevorzugte Verbindungen zur Bildung von polymeren $\chi^{(2)}$ aktiven Materialien sind Verbindungen der Formel Ia, die dadurch gekennzeichnet sind, dass $R^7$ endständig beispielsweise eine der folgenden polymerisierbaren Gruppen trägt:

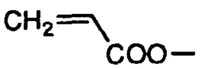 , 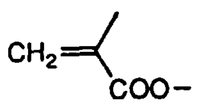 , 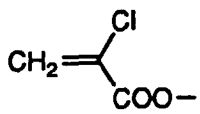 , 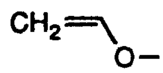 , 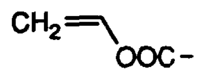

oder

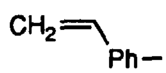

Ebenfalls zur Erfindung gehören demnach LC-Seitenketten-Polymere, die beispielsweise durch Polymerisation von Acrylsäurederivaten, Styrylderivaten und dergleichen, oder durch polymeranaloge Reaktionen von Alkenen an Poly (methylhydrogensiloxanen) hergestellt werden können.

Ueberraschend wurden unter diesen nlo-aktiven ferroelektrischen Flüssigkristallsubstanzen und -substanzmischungen Beispiele gefunden, die bei tiefen Temperaturen in den Glaszustand übergehen und dabei die bei höherer Temperatur in der $S_C$*-Phase induzierte dipolare Ordnung einfrieren. Es ist klar, dass diese niedermolekularen nlo-aktiven ferroelektrischen Flüssigkristallgläser für die technische Anwendung besonders attraktiv sind, da nach dem orientierten Einfrieren des Materials die nlo-Eigenschaften konserviert sind.

Die aus Verbindungen der Formel I, insbesondere der Formel Ia hergestellten LC-Seitenketten Polymere weisen in der Regel die den Monomeren entsprechenden Eigenschaften auf, das heisst sie haben eine ferroelektrische $S_C$*-Phase und nicht linear optische Eigenschaften.

Ein weiterer Anwendungsbereich der vorgestellten Substanzen und Polymere ergibt sich durch die Ausnutzung der Bistabilität in Surface Stabilized Ferroelectric (SSF)-Konfigurationen. Es ist bekannt, dass ferroelektrische Flüssigkristalle mit hinreichend grosser Helixganghöhe in geeignet präparierten Zellen bistabile Schaltzustände einnehmen. Die geforderte grosse Helixganghöhe kann durch Kompensation in ferroelektrischen Flüssigkristallmischungen realisiert werden. In diesem Fall wird eine dipolar geordnete homogene Orientierung, die für die effiziente Erzeugung der Frequenzkonversion günstig ist, durch die Wirkung eines kurzen elektrischen Pulses eingenommen. Ein elektrischer Puls mit entgegengesetzter Polarität schaltet den Film in die nicht-phasenangepasste Orientierung zurück.

Für die vor kurzem entdeckte Surface Bistable Ferroelectric (SBF) Konfiguration gilt dieselbe Anwendungsmöglichkeit. Nur wird in diesem Fall eine kleine Helixganghöhe gefordert. Diese Bedingung erfüllen viele der oben genannten nlo-Substanzen.

Auch nicht-bistabile Konfigurationen, z.B. solche des sog. Deformed Helix Ferroelectric (DHF) -Typs [vgl.Beresnev L.A. et al., Liquid Crystals 5 (1989), 1171] kommen für die erfindungsgemässe Anwendung als $\chi^{(2)}$-aktive Schichten in Betracht. Hier besteht die Möglichkeit, durch Anlegen einer relativ niedrigen Spannung das Material dipolar zu orientieren und damit $\chi^{(2)}$-aktiv zu machen. Diese Orientierung bleibt nur solange erhalten, wie das Feld anliegt. Nach

Abschalten der Spannung relaxiert die Schicht in einen $\chi^{(2)}$-inaktiven Zustand.

Dem Fachmann ist klar, dass die oben beschriebenen Anwendungsmöglichkeiten für die $S_C$*-Phase ebenfalls für die höher geordneten ferroelektrischen Phasen (I*, F*,G*,H*,J*,K*) zutreffen.

Im folgenden werden anhand der beiliegenden Zeichnung Ausführungsformen des erfindungsgemässen optischen Elements beschrieben. Es zeigen

Fig. 1 eine schematische Schnittdarstellung eines Wellenleiterelements,

Fig 2 eine schematische Schnittdarstellung einer alternativen Wellenleiterkonfiguration,

Das in Fig. 1 gezeigte optisch nichtlineare Wellenleiterelement hat den Aufbau einer Flüssigkristallzelle, bestehend aus zwei Glasplatten 1, 7, die mit transparenten Elektroden 2,6 beschichtet sind. Ueber den Elektrodenschichten sind polymere oberflächenbehandelten Claddingschichten 3,5 mit niedrigem Brechungsindex angeordnet. Zwischen diesen beiden derart beschichteten Platten befindet sich ein ferroelektrischer Flüssigkristall 4. Die Dicke der Flüssigkristallschicht ist durch (nicht gezeigte) Abstandshalter fixiert. Die Claddingschichten bewirken die homogene Orientierung der Flüssigkristallschicht und ermöglichen gleichzeitig die Führung der optischen Wellen im Flüssigkristall. In die Claddingschicht 3 ist ein optisches Gitter 8 für die Einkopplung von Licht in die Flüssigkristallschicht 4 und ein weiteres optisches Gitter 9 für die Auskopplung des Lichtes eingeprägt

Bei der Herstellung wird der Raum zwischen den beschichteten Platten mit einer ferroelektrischen Flüssigkristallmischung gefüllt und im elektrischen Feld homogen orientiert. In der ferroelektrischen Phase wird ein Gleichfeld angelegt, welches die lateralen Dipole der ferroelektrischen Flüssigkristallmoleküle senkrecht zur Plattenebene ausrichtet. Durch Abkühlen unter die Glastemperatur der Mischung bei angelegtem Gleichfeld wird die dipolar orientierte Schicht im Glaszustand eingefroren.

In dieses Wellenleiterelement wird Licht einer langwellig emittierenden Laserdiode über das eine der in die Claddingschicht eingeprägten Gitter so eingekoppelt, dass es sich in einer Mode von TE-Charakter als ausserordentlicher Strahl ausbreitet. Bei richtiger Wahl der Schichtdicke und der Ausbreitungsrichtung relativ zur Richtung des Direktors lässt sich eine effektive Ausbreitungskonstante β (ω) der TE-Mode so einstellen, dass die über den nichtlinearen Koeffizienten $d_{21}$ erzeugte 2. Harmonische als TM-ähnliche Welle phasenangepasst die Schicht durchläuft. Am Ende der Zelle werden über das zweite in die Claddingschicht eingeprägte optische Gitter die Grund- und die frequenzverdoppelte Welle ausgekoppelt.

Neben der hier beschriebenen Gittereinkopplung sind auch die aus der Literatur bekannten Verfahren der Prismen- und Stirnkopplung denkbar.

Das in Fig. 2 gezeigte optische Wellenleiterelement besitzt eine Flüssigkristallschicht mit periodisch gepolter Struktur. Es besteht ebenfalls aus zwei Glasplatten 1, 7, die mit transparenten Elektroden 2,6 beschichtet sind. Allerdings weisen die Elektroden eine Gitterstruktur auf, die es erlaubt, die Flüssigkristallschicht räumlich alternierend zu polen. Ueber den Elektrodenschichten sind polymere oberflächenbehandelten Claddingschichten 3,5 mit niedrigem Brechungsindex angeordnet. Zwischen diesen beschichteten Platten befindet sich ein ferroelektrischer Flüssigkristall 4. In die Claddingschicht 3 sind optische Gitter 8, 9 für die Ein- und die Auskopplung von Licht in die bzw. aus der Flüssigkristallschicht 4 eingeprägt

Dabei beträgt die Periode die Länge $2\, l_c = \frac{2\pi}{\Delta\beta}$ mit $\Delta\beta = \beta(2\omega)-2\beta(\omega)$ und ω bedeutet die Kreisfrequenz der Fundamentalwelle, β die Ausbreitungskonstante. Diese Quasi Quasi-Phasematching-Methode erlaubt die Ausnutzung des grössten nlo-Koeffizienten $d_{22}$. Die Lichteinkopplung geschieht wieder mit den im Zusammenhang mit Fig. 1 beschriebenen Methoden.

Die Erfindung wird durch die folgenden Beispiele weiter veranschaulicht. Die optischen Antipoden von chiralen Verbindungen besitzen jeweils die gleichen Phasenumwandlungstemperaturen und absolut gleiche Werte der Verdrillung aber mit entgegengesetzten Vorzeichen.

Die zur Charakterisierung der Phasenumwandlungen verwendeten Abkürzungen stehen für folgende Bedeutungen:

| | |
|---|---|
| C | für kristallin |
| S | für smektisch |
| $S_A$, $S_B$, $S_C$ | etc. für smektisch A, B, C etc. |
| $S_C$*, $S_F$*- | etc. für chiral smektisch C, F etc. |
| N | für nematisch |
| Ch | für cholesterisch |
| I | für isotrop |

Beispiel 1

0,06 g 5-Amino-2-nitro-4-[(R)-2-octyloxy]benzoesäure, 0,1 g 4-(5-Heptyl-2-pyrimidinyl)phenol, 0,04 g 4-(Dimethylamino)pyridin wurden in 10 ml Dichlormethan gelöst und die Lösung innert 10 Minuten unter Rühren portionenweise mit 0,07 g N,N'-Dicyclohexylcarbodiimid versetzt. Das Gemisch wurde 40 Stunden bei Raumtemperatur gerührt, filtriert und eingeengt. Chromatographie des Rückstandes an Kieselgel mit Äthylacetat/Dichlormethan (Vol. 1:8) und Umkristallisation aus Diäthyläther/Hexan (Vol. 1:1) ergab 0,09 g 4-(5-Heptyl-2-pyrimidinyl)phenyl 5-amino-2-nitro-4-[(R)-2-octyloxy]benzoat mit Schmelzpunkt 105°C.

Die als Ausgangsmaterial verwendete 5-Amino-2-nitro-4-[(R)-2-octyloxy]benzoesäure wurde wie folgt hergestellt:

a) Eine Lösung von 10 g 4-Hydroxy-3-nitrobenzoesäure und 370 ml Äthylalkohol wurde vorgelegt und dann wurde Chlorwasserstoffgas während 5 Stunden eingeführt. Das Reaktionsgemisch wurde auf 1300 ml Eiswasser gegossen und der anfallende Feststoff abfiltriert, mit 50%igem Äthylalkohol gewaschen und getrocknet. Dies ergab 8,5 g Äthyl 4-hydroxy-3-nitrobenzoat mit Schmelzpunkt 68,5-69,5°C als gelbe Kristalle.

b) Ein Gemisch von 4 g Äthyl 4-hydroxy-3-nitrobenzoat, 5,4 g Benzolsulfonsäure (S)-2-octylester, 6,9 g Kaliumcarbonat und 110 ml Äthyl-methylketon wurde über Nacht zum Rückfluss erhitzt. Anschliessend wurde das abgekühlte Reaktionsgemisch auf Wasser gegossen und dreimal mit je 50 ml Diäthyläther extrahiert. Die vereinigten organischen Phasen wurden mit 500 ml Wasser gewaschen, über Magnesiumsulfat getrocknet, filtriert und eingeengt. Chromatographie des Rückstandes an Kieselgel mit Dichlormethan ergab 5,6 g Äthyl 3-nitro-4-[(R)-2-octyloxy)benzoat als gelbes Öl.

c) Ein Gemisch von 1,7 g Äthyl 3-nitro-4-[(R)-2-octyloxy]benzoat und 50 ml Äthylalkohol wurde über 0,1 g Platin(II)oxid hydriert, bis die Aufnahme von Wasserstoff beendet wurde. Der Katalysator wurde abfiltriert und das Filtrat eingeengt. Chromatographie des Rückstandes an Kieselgel mit Dichlormethan ergab 1,5 g Äthyl 3-amino-4-[(R)-2-octyloxy]benzoat als farbloses Öl.

d) Eine Lösung von 0,5 g Äthyl 3-amino-4-[(R)-2-octyloxy]benzoat, 3,4 ml Methanol, 1,7 ml Wasser und 0,4 g Kaliumhydroxid wurde während 4 Stunden zum Rückfluss erhitzt. Anschliessend wurde das Methanol zum grössten Teil abdestilliert und das Reaktionsgemisch zuerst mit 50 ml Wasser versetzt und dann mit konzentrierter Schwefelsäure leicht sauer gestellt. Der anfallende Feststoff wurde abfiltriert und getrocknet. Dies ergab 0,44 g 3-Amino-4-[(R)-2-octyloxy]benzoesäure mit $[a]_D^{20}$ = -29,3°.

e) Eine Lösung von 1 g 3-Amino-4-[(R)-2-octyloxy]benzoesäure und 12 ml Essigsäureanhydrid wurde während 1 Stunde bei 90°C erwärmt und dann mit 100 ml Wasser versetzt. Der anfallende Feststoff wurde vom gekühlten Reaktionsgemisch abgetrennt und getrocknet. Dies ergab 0,9 g 3-Acetylamino-4-[(R)-2-octyloxy]benzoesäure.

f) Eine Lösung von 0,4 ml Salpetersäure und 0,3 ml Essigsäure wurde portionenweise bei 0°C mit 0,1 g 3-Acetylamino-4-[(R)-2-octyloxy]benzoesäure versetzt, 10 Minuten bei dieser Temperatur und dann 10 Minuten bei 20°C gerührt und dann auf 6 g Eiswasser gegossen. Der anfallende Feststoff wurde abfiltriert und dann in konzentrierter Kaliumcarbonat-Lösung gelöst. Diese Lösung wurde zweimal mit je 50 ml Essigester extrahiert. Die vereinigten organischen Phasen wurden zweimal mit je 50 ml Wasser gewaschen, über Natriumsulfat getrocknet, filtriert und anschliessend eingeengt. Dies ergab 5-Acetylamino-2-nitro-4-[(R)-2-octyloxy]benzoesäure, welche ohne weitere Reinigung in die nächste Stufe eingesetzt wurde.

In analoger Weise können folgende Verbindungen hergestellt werden:

4-(Nonyloxy)-4'-biphenylyl 5 amino-2-nitro-4-[(R)-2-octyloxy)benzoat, Smp. (C-I) 140-142°C.

4'-[4-(trans-4-Pentylcyclohexyl)butyl]-4-biphenyl 5-amino-2-nitro-4-[(R)-2-octyloxy] benzoat

p-(trans-5-Decyl-m-dioxan-2-yl)phenyl 5-amino-2-nitro-4-[(R)-2-octyloxy] benzoat

p-[trans-5-[(E)-1-Heptenyl]-m-dioxan-2-yl]phenyl 5-amino-2-nitro-4-[(R)-2-octyloxy] benzoat

p-[trans-5-[(E)-1-Heptenyl]-m-dioxan-2-yl]phenyl 5-amino-4-[(R)-2-fluorohexanoyloxy]-2-nitro benzoat

p-[trans-5-[(E)-1-Heptenyl]-m-dioxan-2-yl]phenyl 5-amino-4-[(2S,3S)-2-fluoro-3-methylvaleroyloxy]-2-nitro benzoat

p-[trans-5-[(E)-1-Heptenyl]-m-dioxan-2-yl]phenyl 5-amino-4-[(S)-2-methylbutyloxy]-2-nitro benzoat

4-[5-(trans-5-Heptyl-m-dioxan-2-yl)pyridin-2-yl]phenyl 5-amino-4-[(R)-2-fluorohexanoyloxy]-2-nitro benzoat

5-(p-Octylphenyl)-2-pyrazinyl 5-amino-2-nitro-4[(R)-2-octyloxy] benzoat

5-[p-(trans-4-Pentylcyclohexyl)phenyl]-2-pyrazinyl 5-amino-4-[(S)-2-methylbutyloxy]-2-nitro benzoat

4-[2-(p-Octylphenyl)ethynylphenyl 5-amino-4-[5-hexenoxy-(R)-propionyl-2-oxy]-2-nitro benzoat

4-[2-(p-Octylphenyl)ethynyl]phenyl 5-amino-2-nitro-4-[(R)-2-octyloxy] benzoat

4'-(7-Octenoxy)-4-biphenyl 5-amino-4-[7-octenoxy-(R)-propionyl-2-oxy]-2-nitro benzoat

p-[3-(trans-4-Pentylcyclohexyl)propoxy]phenyl 5-amino-4-[oct-7-enoxy-(R)-propionyl-2-oxy]-2-nitro benzoat

p-[3-(trans-4-Pentylcyclohexyl)propoxy]phenyl 5-amino-4-[oct-3-enoxy-(R)-propionyl-2-oxy]-2-nitro benzoat

Beispiel 2

Ein Gemisch von 5-Dimethylamino-2-nitro-4-[(R)-2-octyloxybenzoesäure, 4-(5-Heptyl-2-pyrimidinyl)phenol, N,N'-Dicyclohexylcarbodiimid, 4-(Dimethylamino)pyridin und Dichlormethan wurde in analoger Weise zu Beispiel 1 umgesetzt. Dies ergab 4-(5-Heptyl-2-pyrimidinyl)phenyl 5-dimethylamino-2-nitro-4-[(R)-2-octyloxy)benzoat als gelbe Kristalle mit Schmelzpunkt 60-62°C und $[\alpha]_D^{20}$ = +3°.

Die als Ausgangsmaterial verwendete 5-Dimethylamino-2-nitro-4-[(R)-2-octyloxy]benzoesäure wurde wie folgt hergestellt:

a) Die Veresterung von 5-Amino-2-nitro-2-[(R)-2-octyloxy]benzoesäure wurde analog zur Synthese von 4-(5-Heptyl-2-pyrimidinyl)phenyl 5-amino-2-nitro-4-[(R)-octyloxy]benzoat durchgeführt. Dies ergab Äthyl 5-amino-2-nitro-4-[(R)-2-octyloxy]benzoat.

b) Ein Gemisch von 0,05 g Äthyl 5-amino-2-nitro-4-[(R)-2-octyloxy]benzoat, 0,13 g Natriumhydrogencarbonat, 2,5 ml Äthylalkohol und 0,5 ml Methyliodid wurde während 72 Stunden zum Rückfluss erhitzt. Das Reaktionsgemisch wurde filtriert und dann eingeengt. Chromatographie des Rückstandes an Kieselgel mit Dichlormethan ergab Äthyl 5-dimethylamino-2-nitro-4-[(R)-2-octyloxy]benzoat, welches ohne weitere Reinigung in die nächste Stufe eingesetzt wurde.

c) Die Verseifung von Äthyl 5-dimethylamino-2-nitro-4-[(R)-2-octyloxy]benzoat wurde analog zur Synthese von 3-Amino-4-[(R)-2-octyloxy]benzoesäure durchgeführt. Dies ergab 5-Dimethylamino-2-nitro-4-[(R)-2-octyloxy]benzoesäure.

Auf analoge Weise können folgende Verbindungen hergestellt werden:

4'-[4-(trans-4-pentylcyclohexyl)butyl]-4-biphenyl 4-[(R)-2-fluorohexanoyloxy]-5-dimethylamino-2-nitro benzoat

4'-[4-(trans-4-pentylcyclohexyl)butyl]-4-biphenyl 5-dimethylamino-4-[(R)-2-fluorohexanoyloxy]-2-nitro benzoat

Beispiel 3

2-Amino-5-nitro-4-[(R)-2-octyloxy]benzoesäure, 4-(5-Heptyl-2-pyrimidinyl)phenol, N,N'-Dicyclohexylcarbodiimid, 4-(Dimethylamino) pyridin und Dichlormethan wurden in analoger Weise zu Beispiel 1 umgesetzt. Dies ergab 4-(5-Heptyl-2-pyrimidinyl)phenyl 2-amino-5-nitro-4-[(R)-2-octyloxy]benzoat mit Schmelzpunkt 105°C mit $[\alpha]_D^{20}$= +19°.

Die als Ausgangsmaterial verwendete 2-Amino-5-nitro-4-[(R)-2-octyloxy]benzoesäure wurde wie folgt hergestellt:

a) Eine Lösung von 20 g 4-Toluidin und 400 g konzentrierter Schwefelsäure (d = 1,84) wurde tropfenweise bei 0°C mit einer Lösung von 15 g Salpetersäure (d = 1,48) und Schwefelsäure (d = 1,84) versetzt. Die Reaktionstemperatur

blieb unter 0°C. Nach Beendung der Zugabe wurde das Reaktionsgemisch 1 Stunde bei 0°C weitergerührt und dann so auf 1200 ml Eiswasser gegossen, dass die Reaktionstemperatur nicht über 25°C anstieg. Das Reaktionsgemisch wurde weiter mit 4000 ml Wasser verdünnt und dann mit Natriumcarbonat-Lösung neutral gestellt. Der anfallende Feststoff wurde abfiltriert, portionenweise mit Wasser gewaschen, getrocknet und dann aus Äthylalkohol umkristallisiert. Dies ergab 23 g 4-Methyl-3-nitroanilin als gelbe Kristalle.

b) Eine Lösung von 10 g 4-Methyl-3-nitroanilin und 40 ml Essigsäureanhydrid wurde während 15 Minuten zum Rückfluss erwärmt. Das abgekühlte Reaktionsgemisch wurde mit 200 ml Wasser versetzt und auf 0°C abgekühlt, dann 30 Minuten bei 0°C gerührt. Der anfallende Feststoff wurde abfiltriert und getrocknet. Dies ergab 11 g 4-Acetylamino-2-nitrotoluol.

c) Eine Suspension von 10 g 4-Acetylamino-2-nitrotoluol, 8 g Magnesiumsulfat und 500 ml Wasser wurde zum Rückfluss erwärmt und dann während 20 Minuten portionenweise mit 24 g Kaliumpermanganat und 8 g Magnesiumsulfat versetzt. Das Reaktionsgemisch wurde während 1,5 Stunden zum Rückfluss erwärmt und dann heiss filtriert, um Restmengen vom Edukt zu entfernen. Das abgekühlte Reaktionsgemisch wurde leicht sauer gestellt und der anfallende Feststoff abfiltriert und getrocknet. Dies ergab 5,6 g 4-Acetylamino-2-nitrobenzoesäure mit Schmelzpunkt 217-220°C.

d) Die Verseifung von 4-Acetylamino-2-nitrobenzoesäure wurde analog zur Synthese von 5-Amino-2-nitro-4-[(R)-2-octyloxy]benzoesäure durchgeführt. Dies ergab 4-Amino-2-nitrobenzoesäure mit Schmelzpunkt 244-245°C.

e) Eine warme Lösung von 3 g 4-Amino-2-nitrobenzoesäure, 5 ml Wasser und 3,6 ml konzentrierter Schwefelsäure wurde auf 0°C abgekühlt, zuerst mit 8,5 g Eiswasser und dann innert 10 Minuten bei 0°C mit einer Lösung von 1,3 g Natriumnitrit und 2,8 ml Wasser versetzt. Das Reaktionsgemisch wurde nach 1 Stunde bei dieser Temperatur gerührt und dann mit 1,8 g Harnstoff versetzt und dann filtriert. Das Filtrat wurde während 10 Minuten zu einer kochenden Lösung von 12 ml konzentrierter Schwefelsäure und 8,3 ml Wasser zugetropft. Das Reaktionsgemisch wurde während 3 Stunden zum Rückfluss erwärmt und dann auf Raumtemperatur abgekühlt. Der anfallende Feststoff wurde abfiltriert. Dies ergab 1 g 4-Hydroxy-2-nitrobenzoesäure als gelbe Kristalle mit Schmelzpunkt 230°C (Zersetzung).

f) Die Veresterung von 4-Hydroxy-2-nitrobenzoesäure wurde analog zur Synthese von Äthyl 4-hydroxy-3-nitrobenzoat durchgeführt. Dies ergab Äthyl 4-hydroxy-2-nitrobenzoat.

g) Die Verätherung von Äthyl 4-hydroxy-2-nitrobenzoat wurde analog zur Synthese von Äthyl 3-nitro-4-[(R)-2-octyloxy]benzoat durchgeführt. Dies ergab Äthyl 2-nitro-4-[(R)-2-octyloxy]benzoat mit $[\alpha]_D^{20}$ =-9,4°.

h) Die Hydrierung von Äthyl 2-nitro-4-[(R)-2-octyloxy]benzoat wurde analog zur Synthese von Äthyl 3-amino-4-[(R)-2-octyloxy]benzoat durchgeführt. Dies ergab Äthyl 2-amino-4-[(R)-2-octyloxy]benzoat als farbloses Öl.

i) Die Verseifung von Äthyl 2-amino-4-[(R)-2-octyloxy]benzoat wurde analog zur Synthese von 3-Amino-4-[(R)-2-octyloxy]benzoesäure durchgeführt. Dies ergab 2-Amino-4-[(R)-2-octyloxy]benzoesäure mit Schmelzpunkt 94-95,5°C.

j) Die Acylierung von 2-Amino-4-[(R)-2-octyloxy]benzoesäure wurde analog zur Synthese von 3-Acetylamino-4-[(R)-2-octyloxy]benzoesäure durchgeführt. Dies ergab 2-Acetylamino-4-[(R)-2-octyloxy]benzoesäure mit $[\alpha]_D^{20}$ =-4.4°.

k) Die Nitrierung von 2-Acetylamino-4-[(R)-2-octyloxy]benzoesäure wurde analog zur Synthese von 5-Acetylamino-2-nitro-4-[(R)-2-octyloxy]benzoesäure durchgeführt. Dies ergab 2-Acetylamino-5-nitro-4-[(R)-2-octyloxy]benzoesäure.

l) Die Verseifung von 2-Acetylamino-5-nitro-4-[(R)-2-octyloxy]benzoesäure wurde analog zur Synthese von 5-Amino-2-nitro-4-[(R)-2-octyloxy]benzoesäure durchgeführt. Dies ergab 2-Amino-5-nitro-4-[(R)-2-octyloxy]benzoesäure.

In analoger Weise können die folgenden Verbindungen hergestellt werden:

4-(Nonyloxy)-4'-biphenylyl 2-Amino-5-nitro-4-[(R)-2-octyloxy]benzoat mit Schmelzpunkt 133-134°C.

4-[5-(trans-5-Heptyl-m-dioxan-2-yl)pyridin-2-yl]phenyl 2-amino-4-[(R)-2-fluorhexanoyloxy]-5-nitro benzoat

4'-(Dec-9-enyloxy)-4-biphenylyl 2-amino-5-nitro-4-[octyloxy-(R)-propionyl-2-oxy] benzoat

p-(5-Heptyl-2-pyrimidinyl)phenyl 2-amino-4-[(S)-2-methylbutyloxy]-5-nitro benzoat

p-Heptyloxyphenyl 2-amino-5-nitro-4-[(R)-2-octyloxy] benzoat

4'-Pentyl-4-biphenylyl 2-amino-5-nitro-4-[(R)-2-octyloxy] benzoat

p-(trans-5-Decyl-m-dioxan-2-yl)phenyl 2-amino-5-nitro-4-[(R)-2-octyloxy] benzoat

p-[2-(trans-4-Pentylcyclohexyl)ethyl]phenyl 2-amino-5-nitro-4-[(R)-2-octyloxy] benzoat

p-[2-(trans-4-Pentylcyclohexyl)ethyl]phenyl 2-amino-4-[(2S,3S)-2-fluor-3-methylvaleroyloxy]-5-nitro benzoat

4'-[4-(trans-4-Pentylcyclohexyl)butyl]-4-biphenyl 2-amino-4-[(R)-2-fluorhexanoyloxy]-5-nitro benzoat

4'-[4-(trans-4-Pentylcyclohexyl)butyl]-4-biphenyl 2-amino-5-nitro-4-[(R)-2-octyloxy] benzoat

p-[3-(trans-4-Pentylcyclohexyl)propoxyl]phenyl 2-amino-4-[(R)-2-fluorhexanoyloxy]-5-nitro benzoat

p-[(trans-4-Pentylcyclohexyl)methoxy]phenyl 2-amino-4-[(R)-2-fluorhexanoyloxy]-5-nitro benzoat

4-[5-(trans-5-Heptyl-m-dioxan-2-yl)pyridin-2-yl]phenyl 2-amino-4-[(R)-2-fluorhexanoyloxy]-5-nitro benzoat

p-(trans-5-[(E)-1-Heptenyl]-m-dioxan-2-yl)phenyl 2-amino-4-[(R)-2-fluorhexanoyloxy]-5-nitro benzoat

4'-(trans-5-[(E)-1-Heptenyl]-m-dioxan-2-yl)-4-biphenyl 2-amino-4-[(R)-2-fluorhexanoyloxy]-5-nitro benzoat

5-[p-(Heptyloxy)phenyl]-2-pyrazinyl 2-amino-5-nitro-4-[(R)-2-octyloxy] benzoat

5-[p-(10-Undecenyloxy)phenyl]-2-pyrazinyl 2-amino-4-[(R)-2-fluorhexanoyloxy]-5-nitro benzoat

5-[p-(trans-4-Pentylcyclohexyl)phenyl]-2-pyrazinyl 2-amino-5-nitro-4-[(R)-2-octyloxy] benzoat

5-[p-(trans-4-Pentylcyclohexyl)phenyl]-2-pyrazinyl 2-amino-4-[(2S,3S)-2-fluoro-3-methylvaleroyloxy]-5-nitro benzoat

5-[p-[(R)-2-octyloxy]phenyl]-2-pyrazinyl 2-amino-5-nitro-4-[(R)-2-octyloxy] benzoat

p-[(E)-4-Octylstyryl]phenyl 2-amino-5-nitro-4-[(R)-2-octyloxy] benzoat

4-[2-(p-Octylphenyl)ethynyl]phenyl 2-amino-5-nitro-4-[(R)-2-octyloxy] benzoat

4'-(10-Undecenoxy)-4-biphenyl 2-dimethylamino-4-[(R)-2-fluorohexanoyloxy]-5-nitro benzoat

4'-[(S)-3-Methylpentyl]-4-biphenyl 2-dimethylamino-4-[(2S,3S)-2-fluoro-3-methylvaleroyloxy]-5-nitro benzoat

Hydrochinon-bis[2-amino-5-nitro-4-[(R)-2-octyloxy] benzoat]

Biphenyl-4,4'-bis[2-amino-5-nitro-4-[(R)-2-octyloxy] benzoat]

Biphenyl-4,4'-bis[2-amino-4-[(R)-2-fluorohexanoyloxy]-5-nitro benzoat]

Beispiel 4

Die Veresterung von 4-[2-Amino-5-nitro-4-[(R)-2-octyloxy]phenyl]benzoat mit 4-Hydroxy-4'-nonyloxybiphenyl wurde analog zur Synthese von 4'-(5-Heptyl-2-pyrimidinyl)phenyl-5-amino-2-nitro-4-[(R)-2-octyloxy]benzoat durchgeführt. Dies ergab 4'-(4-Nonyloxyphenyl)phenyl 4-[2-amino-5-nitro-4-[(R)-2-octyloxy]phenyl]benzoat. Smp. (C-I) 90,1°C, Umwandlung ($S_C$-$S_A$) 132°C, Umwandlung ($S_A$-Ch) 186°C, Klärpunkt (Ch-I) 190,6°C.

Die als Ausgangsmaterial verwendete 4-[2-Amino-5-nitro-4-[(R)-2-octyloxy]phenyl]benzoesäure wurde wie folgt hergestellt:

a) Ein Gasgemisch aus Kohlendioxid und Brom wird in eine auf 120-140°C erhitzte Schmelze von 10,5 g m-Nitrophenol eingeleitet, bis die berechnete Gewichtszunahme erreicht ist. Durch das Einleiten von reinem Kohlendioxid werden Bromreste aus dem Reaktionsgemisch entfernt. Das Produktgemisch wurde zweimal aus 10%iger wässriger Salzsäure umkristallisiert. Dies ergab 8 g 4-Brom-3-nitrophenol.

b) Die Verätherung von 4-Brom-3-nitrophenol wurde analog zur Synthese von Äthyl 3-nitro-4-[(R)-2-octyloxy]benzoat durchgeführt. Dies ergab 1-Brom-2-nitro-4-[(R)-2-octyloxy]benzol.

c) Eine Lösung von 25,4 g 2-(p-Bromphenyl)-4,4-dimethyl-2-oxazolin in 100 ml absolutem Tetrahydrofuran wurde unter Stickstoff 2,7 g Magnesiumspänen zugetropft, so dass das Gemisch durch die Reaktion schwach siedete. Nach beendeter Zugabe wurde weitere 2 Stunden unter Rückfluss erhitzt. Der Umsatz beträgt 95% (gaschromatographisch).

20 ml der Lösung des Grignardreagenzes wurden bei -70°C unter Stickstoffatmosphäre und intensivem Rühren zu einer Mischung aus 10 ml Triisopropylborat und 0,5 ml trockenem Tetrahydrofuran getropft. Nach beendeter Zugabe wurde das Reaktionsgemisch langsam auf Raumtemperatur erwärmt und weitere 30 Minuten gerührt. Danach wurde das Reaktionsgemisch in 100 ml 10%ige Salzsäure gegossen. Die Lösung wurde mit festem Natriumcarbonat auf pH = 9 eingestellt und dreimal mit 100 ml Essigsäureäthylester extrahiert. Die vereinigten organischen Phasen wurden zweimal mit je 100 ml Wasser gewaschen, über Natriumsulfat getrocknet, filtriert und eingeengt. Chromatographie des Rückstandes an Kieselgel mit einem Gemisch aus Essigsäureäthylester und Äthanol (Vol. 2:1) ergab 4-[2-(4,4-Dimethyl-2-oxazolyl)]phenylhoronsäure.

d) Einer Mischung von 0,684 g 1-Brom-2-nitro-4-[(R)-2-octyloxy]benzol, 80 mg $Pd(PPh_3)_4$ und 4,6 ml Toluol wurden 0,6 g 4-[2-(4,4-Dimethyl-2-oxazolyl)]phenylboronsäure in 1,14 ml Methanol und 2,28 ml 2M wässriger Natriumcarbonatlösung zugefügt. Das Reaktionsgemisch wurde unter intensivem Rühren 48 Stunden auf 80°C erhitzt. Nach dem Abkühlen wurden 10 ml 2M wässriger $Na_2CO_3$-Lösung und 1 ml konzentriertes Ammoniakwasser zugefügt und das Reaktionsgemisch mit 20 ml Methylenchlorid extrahiert. Die organische Phase wird abgetrennt, über Magnesiumsulfat getrocknet und eingeengt. Chromatographie des Rückstandes an Kieselgel mit einem Gemisch aus Essigsäureäthylester und Cyclohexan (Vol. 2:3) ergibt schliesslich 0,79 g 2-[2'-nitro-4'-[(R)2-octyloxy]-4-biphenyl]-4,4-dimethyl-2-oxazolin.

e) 0,62 g 2-[2'-nitro-4'-[(R)-2-octyloxy]-4-biphenyl]-4,4-dimethyl-2-oxazolin werden in 15 ml 3N Salzsäure unter Rühren auf 95°C erhitzt. Nach dem Abkühlen wird der Niederschlag abfiltriert und mit 10 ml Wasser gewaschen. Anschliessend wird der Niederschlag in 15 ml 20%iger Natriumhydroxidlösung in Methanol und Wasser (Vol. 1:1) für 40 Minuten unter intensivem Rühren auf 70°C erhitzt. Nach dem Abkühlen wird das Reaktionsgemisch mit konzentrierter Salzsäure neutralisiert. Der angefallene Feststoff wird abfiltriert. Nach dem Trocknen im Vakuum ergab dies 0,48 g 4-[4'-[(R)-2-octyloxy]-2'-nitrophenyl]benzoesäure.

f) Die Reduktion von 4-[4'-[(R)-2-octyloxy]-2'-nitrophenyl]benzoesäure wurde analog zur Synthese von Äthyl 3-amino-4-[(R)-2-octyloxy]benzoat durchgeführt. Dies ergab 4-[2-Amino-4-[(R)-2-octyloxy]phenyl]benzoesäure.

g) Die Acetylierung von 4-[2-Amino-4-[(R)-2-octyloxy]phenyl]benzoesäure wurde analog zur Synthese von 3-Acetylamino-4-[(R)-2-octyloxy]benzoesäure durchgeführt. Dies ergab 4-[2-Acetylamino-4-[(R)-2-octyloxy]phenyl]benzoesäure.

h) Die Nitrierung von 4-[2-Acetylamino-4-[(R)-2-octyl-oxy]phenyl]benzoesäure wurde analog zur Synthese von 5-Acetylamino-2-nitro-4-[(R)-2-octyloxy]benzoesäure durchgeführt. Dies ergab 4-[2-Acetylamino-5-nitro-4-[(R)-2-octyloxy]phenyl]benzoesäure.

i) Die Verseifung der Amidfunktion von 4-[2-Acetylamino-5-nitro-4-[(R)-2-octyloxy]phenyl]benzoesäure wurde analog zur Synthese von 5-Amino-2-nitro-4-[(R)-2-octyloxy]benzoesäure durchgeführt. Dies ergab 4-[2-Amino-5-nitro-4-[(R)-2-octyloxy]phenyl]benzoesäure.

In analoger Weise können folgende Verbindungen hergestellt werden:

4'-(5-Heptyl-2-pyrimidinyl)phenyl 4-[2-amino-5-nitro-4-[(R)-2-octyloxy]phenyl]benzoat. Smp. (C-I) 175-176,1°C.

4-Heptyloxyphenyl 4-[2-amino-5-nitro-4-[(R)-2-octyloxy]phenyl]benzoat. Smp. (C-I) 95-96,1°C, Umwandlung ($S_A$-I) 74°C.

4'-(4-Pentylphenyl)phenyl 4-[2-amino-5-nitro-4-[(R)-2-octyloxy]phenyl]benzoat. Smp. (C-$S_A$) 142°C, Umwandlung ($S_A$-Ch) 160,8°C, Umwandlung (Ch-I) 168,1°C.

4-[2-(trans-4-Pentylcyclohexyl)ethyl]phenyl 4-[2-amino-5-nitro-4-[(R)-2-octyloxy]phenyl]benzoat. Smp. (C-$S_A$) 110°C, Umwandlung ($S_A$-Ch) 132°C, Umwandlung (Ch-I) 139°C, monotrope Umwandlung ($S_C$*-$S_A$) 62°C.

1,4-Phenylen-bis[4-[2-amino-5-nitro-4-[(R)-2-octyloxy]phenyl]benzoat. Smp. (C-$S_A$) 175°C, Umwandlung ($S_A$-Ch) 186°C, Umwandlung (Ch-I) 201°C.

<u>Beispiel 5</u>

Die Veresterung von 4-[5-Amino-2-nitro-4-[(R)-2-octyloxy]phenyl]benzoesäure mit 4-Hydroxy-4'-octyloxybipbenyl wird analog zu 4'-(5-Heptyl-2-pyrimidinyl)phenyl-5-amino-2-nitro-4-[(R)-2-octyloxy]benzoat durchgeführt. Dies ergab 4'-(Octyloxy)-4-biphenylyl-4-[5-amino-2-nitro-4[(R)-2-octyloxy]phenyl]benzoat. Smp. (C-I) 177,8-180,5°C, Umwandlung ($S_A$-Ch) 128°C, Klp. (Ch-I) 150°C.

Die als Ausgangsmaterial verwendete 4-[5-Amino-2-nitro-4-[(R)-2-octyloxy]phenyl]benzoesäure wurde wie folgt hergestellt:

a) 4-[4-Hydroxyphenyl]benzoesäure wurde mit Äthanol analog zur Synthese von 4-Hydroxy-3-nitrobenzoesäureäthylester hergestellt. Dies ergab Äthyl 4-[4-hydroxyphenyl]benzoat.

b) Die Verätherung von Äthyl 4-[4-hydroxyphenyl]benzoat wurde analog zur Synthese von Äthyl 3-nitro-4-[(R)-2-octyloxy]benzoat durchgeführt. Dies ergab Äthyl 4-[4-[(R)-2-octyloxy]phenyl]benzoat.

c) Die Verseifung von Äthyl 4-[4-[(R)-2-octyloxy]phenyl]benzoat wurde analog zur Synthese von 3-Amino-4-[(R)-2-octyloxy]benzoesäure durchgeführt. Dies ergab 4-[4-[(R)-2-octyloxy]phenyl]benzoesäure.

d) 3 g 4-[4-[(R)-2-octyloxy]phenyl]benzoesäure werden bei 95°C in 110 ml Eisessig gelöst. Dieser Lösung wird tropfenweise eine Mischung aus 9 ml rauchender Salpetersäure und 9 ml Eisessig hinzugefügt. Das Reaktionsgemisch wird unter Rühren noch 1 Stunde auf 90-95°C erhitzt und abschliessend 10 Minuten zum Sieden erhitzt. Dabei wurden ca. 40 ml Essigsäure abdestilliert. Das auf Raumtemperatur abgekühlte Reaktionsgemisch wurde auf 250 ml Eiswasser gegossen und der anfallende Feststoff abfiltriert. Umkristallisation aus 50%igem Äthylalkohol ergab 2,6 g 4-[3-Nitro-4-[(R)-2-octyloxy]phenyl]benzoesäure.

e) Die Reduktion von 4-[3-Nitro-4-[(R)-2-octyloxy]phenyl]benzoesäure wurde analog zur Synthese von Äthyl 3-amino-4-[(R)-2-octyloxy]benzoat durchgeführt. Dies ergab 4-[3-Amino-4-[(R)-2-octyloxy]phenyl]benzoesäure.

f) Die Acetylierung der Aminogruppe von 4-[3-Amino-4-[(R)-2-octyloxy]phenyl]benzoesäure wurde analog zur Synthese von 3-Acetylamino-4-[(R)-2-octyloxy]benzoesäure durchgeführt. Dies ergab 4-[3-Acetylamino-4-[(R)-2-octyloxy]phenyl]benzoesäure.

g) 220 mg 4-[3-Acetylamino-4-[(R)-2-octyloxy]phenyl]benzoesäure wird in mehreren Portionen in 2,8 ml einer eisgekühlten Mischung aus Salpetersäure/Eisessig (Vol. 2:5) gegeben. Nach vollständiger Zugabe wird das Reaktionsgemisch weitere 20 Minuten bei 0°C gerührt und anschliessend auf 100 ml Eiswasser gegossen. Der ausgefallene Feststoff wird abfiltriert und mit Wasser gewaschen. Die so erhaltene 4-[5-Acetylamino-2-nitro-4-[(R)-2-octyloxy]phenyl]benzoesäure wird ohne weitere Reinigung in die Folgereaktion eingesetzt.

h) Die Verseifung der Acetylaminogruppe von 4-[5-Acetylamino-2-nitro-4-[(R)-2-octyloxy]phenyl]benzoesäure wurde analog zur Synthese von 5-Amino-2-nitro-4-[(R)-2-octyloxy]benzoesäure durchgeführt. Dies ergab 160 mg 4-[5-Amino-2-nitro-4-[(R)-2-octyloxy]phenyl]benzoesäure.

In analoger Weise können folgende Verbindungen hergestellt werden:

4'-[2-(trans-4-Pentylcyclohexyl)ethyl]phenyl 4-[5-amino-2-nitro-4-[(R)-2-octyl]phenyl]benzoat. Smp. (C-I) 166,5-167,6°C.

4'-(4-Pentylphenyl)phenyl 4-[5-amino-2-nito-4-[(R)-2-octyl]phenyl]benzoat. Smp. (C-I) 187,2-188,3°C.

4'-Heptyloxyphenyl 4-[5-amino-2-nitro-4-[(R)-2-octyl]phenyl]benzoat. Smp. (C-I) 136,5-138,9°C.

Beispiel 6

Herstellung von Poly{4-(11-Acryloyloxyundecanoxy)phenyl 4-[2-amino-5-nitro-4-[(R)-2-octyloxy]phenyl]benzoat}: Eine Lösung von 1 g 4-(11-Acryloyloxyundecanoxy)phenyl 4-[2-amino-5-nitro4-[(R)-2-octyloxy]phenylbenzoat und 2,4 mg $\alpha,\alpha'$-Azo-isobutyronitril in 2,8 ml Toluol wird bei 0°C 10 Min. mit Argon durchströmt und anschliessend im geschlossenen Gefäss 48 Stunden auf 70°C erhitzt. Nach Abkühlen des Polymerisationsgemisches auf Raumtemperatur wird das ausgefallene Polymer abfiltriert und in 10 ml Tertahydrofuran gelöst. Die Lösung wird langsam in 300 ml Methanol getropft, wobei das Polymer ausfällt. Dieser Löse-/Fällungsvorgang wird weitere viermal wiederholt, das Filtrat wird im Vakuum getrocknet und ergibt 0,57 g Poly{4-(11-Acryloyloxyundecanoxy)phenyl 4-[2-amino-5-nitro-4-[(R)-2-otyloxy]phenyl]benzoat}. Glasübergang: 55°C, Umwandlung ($S_C$*-$S_A$): 109°C, Umwandlung ($S_A$-I): 128°C.

Das als Ausgangsmaterial verwendete Monomere 4-(11-Acryloyloxyundecanoxy)phenyl 4-[2-amino-5-nitro-4-[(R)-2-octyloxy] phenyl]benzoat wurde wie folgt hergestellt:

a) 4-(11-Hydroxyundecanoxy)phenol: Ein Gemisch aus 5 g 11-Bromundecanol und 5 g Hydrochinon in 200 ml 2-Butanon, sowie 4 g Kaliumcarbonat wird 48 Stunden unter Rückfluss erhitzt. Das Reaktionsgemisch wird filtriert und das Lösungsmittel abdestilliert. Der in 400 ml Essigester gelöste Rückstand wird viermal mit je 100 ml einer 10 % (w/v) wässr. Kaliumcarbonat und anschliessend zweimal mit Wasser gewaschen. Die Lösung wird über Natriumsulfat getrocknet, filtriert und eingedampft. Umkristallisation aus wenig Essigester ergibt. 4 g 4-(11-Hydroxyundecanoxy)phenol. Smp.: 99-100,5°C

b) 4-(11-Acryloyloxyundecanoxy)phenol: 5 g 4-(11-Hydroxyundecanoxy)phenol, 12 g Acrylsäure und 0,85 g p-Toluolsulfonsäure werden in 250 ml Chloroform gelöst. Das Reaktionsgemisch wird 48 Stunden an einem Wasserabscheider unter Rückfluss erhitzt. Nach dem Abkühlen wird das Reaktionsgemisch zweimal mit je 100 ml einer 10 % (w/v) Natriumhydrogencarbonatlösung und anschliessend dreimal mit je 100 ml Wasser gewaschen. Nach dem Trocknen der organischen Phase über Natriumsulfat wird das Chloroform abdestilliert, das ergibt 5,2 g 4-(11-Acryloyloxyundecanoxy)phenol. Smp. 72 - 74°C

c) 4-(11-Acryloyloxyundecanoxy)phenyl 4-[2-amino-5-nitro-4-[(R)-2-octyloxy] phenyl]benzoat: Die Veresterung von 4-[2-amino-5-nitro-4 [(R)-2-octyloxy]phenyl]benzoesäure mit 4-(11-Acryloyloxyundecanoxy)phenol wurde analog zur Synthese von 4'-(5-Heptyl-2-pyrimidinyl)phenyl [5-amino-2-nitro-4-[(R)-2-octyloxy]phenyl] benzoat (Beispiel 3) durchgeführt. Dies ergab 2,6 g 4-(11-Acrloyloxyundecanoxy)phenyl 4-[2-amino-5-nitro-4-[(R)-2-octyloxy]phenyl]benzoat. Umwandlung (KI - KII) 56,5 - 58°C, Smp. (KII - I) 68°C

Folgende Momomere können auf analoge Weise hergestellt werden:

4-(11-Acryloyloxyundecanoxy)phenyl 4-[2-amino-5-nitro-4-[(R)-2-octyloxy]phenyl]benzoat
4-(11-Methacryloyloxyundecanoxy)phenyl 4-[2-amino-5-nitro-4-[(R)-2-octyloxy]phenyl]benzoat
4-[11-(2-Chloracryloyloxy)undecanoxy]phenyl 4-[2-amino-5-nitro-4-[(R)-2-fluorohexanoyloxy]phenyl]benzoat
4-(11-Vinyloxyundecanoxy)phenyl 4-[2-amino-5-nitro-4-[(R)-2-octyloxy]-phenyl]benzoat
Poly{4-(10-vinyloxycarbonyldecanoxy)phenyl 4-[2-amino-5-nitro-4-[(R)-2-octyloxy]phenyl]benzoat}
4-[11-(4-Styryl)oxyundecanoxy]phenyl 4-[2-amino-5-nitro-4-[(R)-2-octyloxy]phenyl]benzoat
4-[11-(4-styryl)oxyundecanoxy]phenyl 4-[5-amino-4-[(2S,3S)-2-fluoro-3-methylvaleroyloxy]-2-nitrophenyl]benzoat
4-[11-(4-Styryl)methylenoxyundecanoxy]phenyl 4-[2-amino-5-nitro-4-[(R)-2-octyloxy]phenyl]benzoat
4-(10-Undecenyloxy)phenyl 4-[2-N,N-dimethylamino-5-nitro-4-[(R)-2-octyloxy]phenyl]benzoat
4-(11-Acryloyloxyundecanoxy)phenyl 4-[5-amino-2-nitro-4-[(R)-2-octyloxy]phenyl]benzoat

Daraus können in analoger Weise folgende Polymere hegestellt werden:

Poly{4-(11-acryloyloxyundecanoxy)phenyl 4-[2-amino-5-nitro-4-[(R)-2-octyloxy]phenyl]benzoat}

Poly{4-(11-methacryloyloxyundecanoxy)phenyl 4-[2-amino-5-nitro-4-[(R)-2-octyloxy]phenyl]benzoat}

Poly{4-[11-(2-chloracryloyloxy)undecanoxy]phenyl 4-[2-amino-5-nitro-4-[(R)-2-fluorohexanoyloxy]phenyl]benzoat}

Poly{4-(11-vinyloxyundecanoxy)phenyl 4-[2-amino-5-nitro-4-[(R)-2-octyloxy]phenyl]benzoat}

Poly{4-(10-vinyloxycarbonyldecanoxy)phenyl 4-[2-amino-5-nitro-4-[(R)-2-octyloxy]phenyl]benzoat}

Poly{4-[11-(4-styryl)oxyundecanoxy]phenyl 4-[2-amino-5-nitro-4-[(R)-2-octyloxy]phenyl]benzoat}

Poly{4-[11-(4-styryl)oxyundecanoxy]phenyl 4-[5-amino-4-[(2S,3S)-2-fluoro-3-methylvaleroyloxy]-2-nitrophenyl] benzoat}

Poly{4-[11-(4-styryl)methylenoxyundecanoxy]phenyl 4-[2-amino-5-nitro-4-[(R)2-octyloxy]phenyl]benzoat}

Poly{methyl [p-undecyloxyphenyl 4-[2-N,N-dimethylamino-5-nitro-4-[(R)-2-octyloxy]phenyl]benzoat] siloxan}

Poly{4-(11-acryloyloxyundecanoxy)phenyl 4-[5-amino-2-nitro-4-[(R)-2-octyloxy]phenyl]benzoat}

Beispiel 7

Herstellung von Poly{4'-(11-acryloyloxyundecanoxy)-4-biphenyl 4-[2-amino-5-nitro-4- [(R)-2-octyloxy]phenyl]benzoat}: Die Polymerisation von 4'-(11-Acryloyloxyundecanoxy)-4-biphenyl 4-[2-amino-5-nitro-4-[(R)-2-octyloxy]phenyl] benzoat} wurde analog zur Polymerisation von 4-(11-acryloyloxyundecanoxy)phenyl 4-[2-amino-5-nitro-4-[(R)-2-octyloxy]phenyl]benzoat} durchgeführt. Dies ergab 0,63 g Poly{4'-(11-acryloyloxyundecanoxy)-4-biphenyl 4-[2-amino-5-nitro-4-[(R)-2-octyloxy]phenyl]benzoat}. Glasübergang: 90°C Umwandlung ($S_C$* - $S_A$ ): ca. 140°C, Umwandlung ($S_A$ - T): 270°C.

Das als Ausgangsmaterial verwendete Monomere 4'-(11-Acryloyloxyundecanoxy)-4-biphenyl 4-[2-amino-5-nitro-4-[(R)-2-octyloxy]phenyl]benzoat wurde wie folgt hergestellt:

a) 4-Hydroxy-4'-(11-Hydroxyundecanoxy)biphenyl: Die Synthese von 4-Hydroxy-4'-(11-Hydroxyundecanoxy)biphenyl wurde analog zur Synthese von 4-(11-Hydroxyundecanoxy)phenol (Beispiel 6a) durchgeführt. Dies ergab 4-Hydroxy-4'-(11-Hydroxyundecanoxy)biphenyl. Smp.: 144 -146°C

b) 4'-(11-Acryloyloxyundecanoxy)-4-hydroxybiphenyl: Die Veresterung von 4-Hydroxy-4'-(11-Hydroxyundecanoxy) biphenyl wurde analog zur Veresterung von 4-(11-Hydroxyundecanoxy)phenol (Beispiel 6b) durchgeführt. Dies ergab 4'-(11-Acryloyloxyundecanoxy)-4-hydroxybiphenyl. Smp.: 91 - 92°C

c) 4'-(11-Acryloyloxyundecanoxy)-4-biphenyl 4-[2-amino-5-nitro-4-[(R)-2-octyloxy]phenyl]benzoat: Die Veresterung von 4'-(11-Acryloyloxyundecanoxy)-4-hydroxybiphenyl mit 4-[2-amino-5-nitro-4-[(R)-2-octyloxy]phenyl]benzoesäure wurde analog zur Synthese von 4-(11-Acryloyloxyundecanoxy)phenyl 4-[2-amino-5-nitro-4-[(R)-2-octyloxy]phenyl]benzoat (Beispiel 6c) durchgeführt. Dies ergab 4'-(11-Acryloyloxyundecanoxy)-4-biphenyl 4-[2-amino-5-nitro-4-[(R)-2-octyloxy]phenyl]benzoat.,Smp.: 110°C, Umwandlung ($S_A$ - I): 137°C, monotrope Umwandlung ($S_C$* - $S_A$): 84°C

Auf analoge Weise können folgende Monomere hergestellt werden:

4'-(11-Acryloyloxyundecanoxy)-4-biphenylyl 4-[2-amino-5-nitro-4-[(R)-2-octyloxy]phenyl]benzoat

4'-(11-Acryloyloxyundecanoxy)-4-biphenylyl 2-amino-5-nitro-4-[(R)-2-octyloxy] benzoat

4'-(11-Acryloyloxyundecanoxy)-4-biphenylyl 5-amino-2-nitro-4-[(R)-2-octyloxy] benzoat

p-[trans-5-[10-(2-Chloracryloyloxy)decyl]-m-dioxan-2-yl]phenyl 2-amino-5-nitro-4-[(R)-2-octyloxy]benzoat

5-(p-Undec-10-enyloxyphenyl)-2-pyrazinyl 2-N,N-dimethylamino-4-[(R)-2-fluorohexanoyloxy]-5-nitro benzoat

4'-(11-Methacryloyloxyundecanoxy)-4-biphenylyl 2-amino-5-nitro-4-[(R)-2-octyloxy]benzoat

4'-[8-(2-Chloracryloyloxy)octyloxy]-4-biphenylyl 2-amino-6-methyl-4-[(R)-2-fluorohexanoyloxy]-5-nitro benzoat

[p-(11-Acryloyloxyundecyl)-2-primidinyl]phenyl 2-amino-6-fluoro-4-[(R)-2-fluorohexanoyloxy]-5-nitro benzoat

2-Amino-4-[(2S,4R,5S)-4-methy-5-octyl-m-dioxan-2-yl]-5-nitrophenyl trans-4-[p-[8-(2-chloracryloyloxy)octyloxy] phenyl]cyclohexyl]methyl ether

(E)-p-[2-amino-4-[(R)-2-fluorohexanoyloxy]-5-nitrostyryl]phenyl 4-(10-methacryloyloxydecyl)benzoat

Auf analoge Weise können aus obengenannten Monomeren folgende Polymere hergestellt werden:

Poly{4'-(11-acryloyloxyundecanoxy)-4-biphenylyl 4-[2-amino-5-nitro-4-[(R)-2-octyloxy]phenyl]benzoat}

Poly{4'-(11-acryloyloxyundecanoxy)-4-biphenylyl 2-amino-5-nitro-4-[(R)-2-octyloxy] benzoat}

Poly{4'-(11-acryloyloxyundecanoxy)-4-biphenylyl 5-amino-2-nitro-4-[(R)-2-octyloxy] benzoat)

Poly{p-[trans-5-[10-(2-chloracryloyl)oxydecyl]-m-dioxan-2-yl]phenyl 2-amino-5-nitro-4-[(R)-2-octyloxy]benzoat}

Poly{methyl[5-(p-undecyloxyphenyl)-2-pyrazinyl 2-N,N-dimethylamino-4-[(R)-2-fluorhexanoyloxy]-5-nitro benzoat]siloxan}

Poly{4-(11-methacryloyloxyundecanoxy)phenylyl 4-[2-amino-5-nitro-4-[(R)-2-octyloxy]phenyl]benzoat-co-4'-(11-methacryloyl-oxyundecanoxy)-4-bipenylyl 2-amino-5-nitro-4-[(R)-2-octyloxy]benzoat} (1:1)

Poly{4'-[8-(2-chloracryloyloxy)octyloxy]biphenylyl 2-amino-6-methyl-4-[(R)-2-fluorhexanoyloxy]-5-nitro benzoat}

Poly{[p-(11-acryloyloxyundecyl)-2-primidinyl]phenyl 2-amino-6-fluoro-4-[(R)-2-fluorohexanoyloxy]-5-nitro benzoat}

Poly{2-amino-4-[(2S,4R,5S)-4-methyl-5-octyl-m-dioxan-2-yl]-5-nitrophenyl [trans-4-[p-[8-(2-chloracryloyloxy)octyloxy]phenyl]cyclohexyl]methyl ether} and

Poly{(E)-p-[2-amino-4-[(R)-2-fluorohexanoyloxy]-5-nitrostyryl]phenyl 4-(10-methacryloyloxydecyl)benzoat}.

## Patentansprüche

1. Optisches Element zur effizienten Erzeugung kurzwelligen Laserlichtes durch Frequenzkonversion mit einer wellenleitenden Schicht aus einem flüssigkristallinen Material, das eine periodische Struktur aufweist, die eine Quasi-Phasenanpassung eines geführten Laserstrahls erlaubt, wobei die Periodenlänge der räumlichen Struktur durch die Kohärenzlänge $l_c = \pi/\Delta\beta$ des Materials definiert ist, worin $\Delta\beta = \beta_0 (2\,\omega) - 2\,\beta_0 (\omega)$ ist, mit $\omega$ = Kreisfrequenz der Fundamentalwelle, 0 = Mode nullter Ordnung und $\beta$ = Ausbreitungskonstante der Mode,
dadurch gekennzeichnet,

   dass die Periodenlänge der räumlichen Struktur dem Doppelten der Kohärenzlänge $l_c$ entspricht

   und dass das flüssigkristalline Material

   entweder eine ferroelektrische, $\chi^{(2)}$-aktive Mischung chiraler Moleküle mit einer nlo-Molekülgruppe, deren $\chi^{(2)}$-aktive Achse im wesentlichen quer zur Längsachse des chiralen Moleküls gerichtet ist, und $S_c$-Phasen bildenden Flüssigkristallmolekülen,

   oder ein $\chi^{(2)}$-aktives, $S_c$*-Phasen bildendes Seitenkettencopolymer, welches chirale Seitenketten mit einer

nlo-Molekülgruppe besitzt, deren $\chi^{(2)}$-aktive Achse im wesentlichen quer zur Längsachse der Seitenkette gerichtet ist, enthält.

**2.** Optisches Element nach Anspruch 1, dadurch gekennzeichnet, dass das Material aufgrund seiner Doppelbrechungseigenschaft Phasenanpassung der $d_{21}$ - Komponente des nlo-Tensors erlaubt.

**3.** Optisches Element nach einem der Ansprüche 1 - 2, dadurch gekennzeichnet, dass die quer zur Längsachse eingebautenlo-Molekülgruppe ein Nitroanilinderivat ist.

**4.** Optisches Element nach einem der Ansprüche 1 - 3, dadurch gekennzeichnet, dass das ferroelektrische Flüssigkristall - Material eine $S_C$*-Phase oder eine höher geordnete ferroelektrische Phase bildet, die im elektrischen Feld dipolar orientiert werden kann.

**5.** Optisches Element nach einem der Ansprüche 1 - 3, dadurch gekennzeichnet, dass das ferroelektrische LC-Material eine $S_C$*-Phase bildet, die durch Randkräfte bistabil homogen orientiert wird.

**6.** Optisches Element nach Anspruch dadurch gekennzeichnet, dass die dipolare Orientierung durch Abkühlen unter die Glastemperatur fixiert ist.

**7.** Optisches Element nach einem der Ansprüche 1-2, dadurch gekennzeichnet, dass es durch Ausnutzung des Pokkelseffekts als elektrooptischer Schalter oder Lichtmodulator im Spektralbereich von 1300 bis 430 nm arbeitet.

**8.** Optisches Element nach einem der Ansprüche 1-3, dadurch gekennzeichnet, dass die Mischung die Phasenfolge I-Chol-$Sm_A$-$S_C$*-Glas aufweist.

**9.** Optisches Element nach einem der Ansprüche 1 - 2, dadurch gekennzeichnet, dass das Element in der $S_C$*-Phase betrieben wird, derart, dass bei Anlegen eines elektrischen Feldes die $\chi^{(2)}$-Eigenschaft wirksam wird und bei Ausschalten des elektrischen Feldes verschwindet.

**10.** Optisches Element nach einem der Ansprüche 1 - 2, dadurch gekennzeichnet, dass es zur Frequenzverdopplung von Laserlicht der Wellenlänge 850-1300 nm einsetztbar ist.

**11.** Optisches Element nach einem der Ansprüche 4 - 6, dadurch gekennzeichnet, dass die dipolare Orientierung senkrecht auf der Wellenleiterebene steht.

**12.** Frequenzverdoppelndes Modul bestehend aus einer Laserdiode mit Emission im Bereich von 850-1300 nm optisch gekoppelt an ein optisches Element nach einem der vorangehenden Ansprüche.

**13.** Verbindungen der allgemeinen Formel

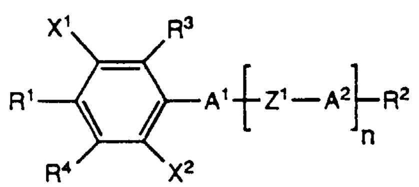

I

mit

$X^1$, $X^2$: eines $-NO_2$ und das andere $-NR^5R^6$, worin $R^5$ und $R^6$ unabhängig voneinander H oder Niederalkyl darstellen;

$R^3$, $R^4$ : unabhängig voneinander H, F, Cl, Br, $NH_2$, $NO_2$, CN, Niederalkyl oder Niederalkoxy, mit der Massgabe, dass $X^1$ verschieden von $R^4$ und $X^2$ verschieden von $R^3$ ist;

$R^1$: chirales Alkyl oder Cycloalkyl worin, unabhängig voneinander, eine oder zwei nicht benachbarte $-CH_2$-Gruppen durch O, S, -COO-, -OOC-, -HC=CH-, und im aliphatischen Rest auch durch -C≡C- ersetzt

sein können, und welches einfach oder mehrfach substituiert sein kann mit F, Cl, CN, Niederalkyl und Niederalkoxy, mit der Massgabe, dass sich das Chiralitätszentrum in einer Alkylkette am ersten oder zweiten C-Atom befindet und bei einem Cycloalkylrest an einem Ring-C-Atom befindet;

$A^1$: 1 bis 4 sechsgliedrige Ringe, welche miteinander bzw. mit dem p-Nitro-Anilinring direkt verknüpft sind und/oder an einer oder gegebenenfalls mehreren Stellen über $-CH_2-CH_2-$, $-CH_2O-$, $-OCH_2-$, -COO-, -OOC-, -COS-, -SOC-, -CH=CH-, -C≡C-, -N=N-, -N=NO-, -ON=N-, -CH=N-, -N=CH-, oder Butylen, worin eine $-CH_2CH_2$-Gruppe durch -COO-, -OOC-, -HC=CH-, -C≡C- und/oder eine $-CH_2$-Gruppe durch O oder S ersetzt sein kann, verknüpft sind. Die sechsgliedrigen Ringe in $A^1$ bedeuten unabhängig voneinander unsubstituiertes oder gegebenenfalls mit F, Cl, Niederalkyl oder Niederalkoxy mono-, di-, oder poly-substituiertes 1,4-Phenylen, in welchem 1 oder 2 -CH-Gruppen durch Stickstoff ersetzt sein können, oder 1,4-Cyclohexylen, in welchem eine oder zwei $-CH_2$- Gruppen (vorzugsweise nicht benachbarte) durch O oder S oder eine $-CH_2CH_2$- Gruppe durch -CH=CH- ersetzt sein können, oder Naphthalin-2,5-diyl, Decalin-2,6-diyl, Tetralin-2,6-diyl, Thiadiazolyl oder Oxodiazolyl;

Z1: eine einfache Kovalenzbindung, $-CH_2-CH_2-$, $-CH_2O-$, $-OCH_2-$, -COO-, -OOC-, -COS-, -SOC-, -CH=CH-, -C≡C-, -N=N-, -N=NO-, -ON=N-, -CH=N-, -N=CH- oder Butylen, worin eine $-CH_2CH_2$-Gruppe durch -COO-, -OOC-, -HC=CH-, -C≡C- und/oder eine $-CH_2$-Gruppe durch O oder S ersetzt sein können;

$A^2$: das Strukturelement

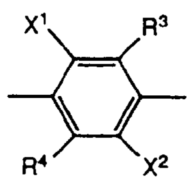

II

worin $X^1$, $X^2$, $R^3$ und $R^4$ die vorher beschriebene Bedeutung haben;

n: die Zahl 0 oder 1;

$R^2$: chirales oder achirales Alkyl oder Cydoalkyl worin, unabhängig voneinander, eine oder mehrere $-CH_2$-Gruppen durch O, S, -COO-, -HC=CH-, und im aliphatischen Rest auch durch -C≡C- oder 1,4 Phenylen ersetzt sein können, und welche einfach oder mehrfach substituiert sein können mit F, Cl, CN, Niederalkyl und Niederalkoxy, mit der Massgabe, dass sich ein allfällig vorhandenes Chiralitäts-zentrum in einer Alkylkette am ersten oder zweiten C-Atom befindet und bei einem Cycloalkylrest an einem Ring-C-Atom befindet, oder falls n die Zahl 0 bedeutet, auch die Gruppe

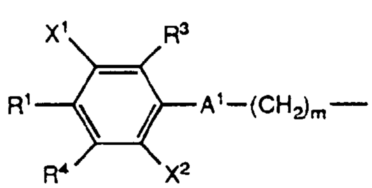

III

worin $X^1$, $X^2$, $R^1$, $R^3$, $R^4$ und $A^1$ obige Bedeutung haben und m eine ganze Zahl 6-16 bedeutet, worin eine oder zwei nicht benachbarte $-CH_2$-Gruppen unabhängig voneinander durch O, S, -COO-, -CH=CH- oder -C≡C- ersetzt sein können.

**14.** Verbindungen der Formel I gemäss Anspruch 13, dadurch gekennzeichnet, dass $R^2$ chirales oder achirales Alkyl oder Cycloalkyl bedeutet, worin, unabhängig voneinander, eine oder zwei nicht benachbarte $-CH_2$- Gruppen durch -O-, -S-, -COO-, -HC=CH-, und im aliphatischen Rest auch -C≡C- ersetzt sein können, und welches einfach oder

mehrfach substituiert sein kann mit F, Cl, CN, Niederalkyl und Niederalkoxy, mit der Massgabe, dass sich ein allfällig vorhandenes Chiralitätszentrum in einer Alkylkette am ersten oder zweiten C-Atom befindet und in einem Cycloalkylrest an einem Ring-C-Atom befindet, oder falls n die Zahl 0 bedeutet auch die Gruppe

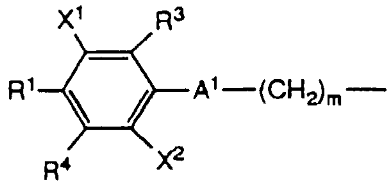

III

worin $X^1$, $X^2$, $R^1$, $R^3$, $R^4$ und $A^1$ obige Bedeutung haben und m eine ganze Zahl 6-16 bedeutet, worin eine oder zwei nicht benachbarte $-CH_2$-Gruppen unabhängig voneinander durch O, S, -COO-, -CH=CH- oder -C≡C- ersetzt sein können.

**15.** Nicht linear optische Verbindungen gemäss Anspruch 13 der allgemeinen Formel

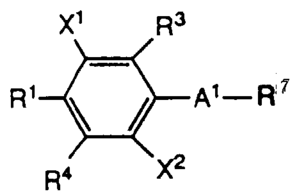

Ia

worin $R^7$ chirales oder achirales Alkyl, in welchem, unabhängig voneinander, eine oder mehrere $-CH_2$-Gruppen durch -O-, -S-, -COO-, -HC=CH-, -C≡C- oder 1,4-Phenylen ersetzt sein können, und welches einfach oder mehrfach substituiert sein kann mit F, Cl, CN, Niederalkyl und Niederalkoxy, bedeutet.

**16.** Nicht linear optische Verbindungen gemäss einem der Ansprüche 13 oder 15, dadurch gekennzeichnet, dass $R^2$ oder $R^7$ endständig eine der folgenden polymerisierbaren Gruppen trägt:

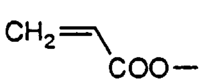 , 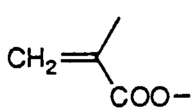 , 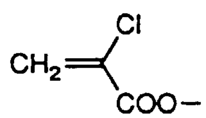 , 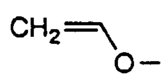 , 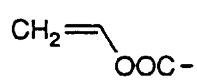

oder

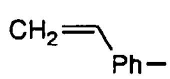

**17.** Verwendung der nicht linearen optischen Verbindungen gemäss einem der Ansprüche 15 oder 16 zur Bildung nicht linearer optischer Polymere.

**18.** Verwendung der nicht linearen optischen Verbindungen gemäss einem der Ansprüche 13 bis 16 in einem optischen Element gemäss einem der Ansprüche 1-11.

**19.** Verwendung der nicht linearen optischen Polymere gemäss Anspruch 17 in einem optischen Element gemäss einem der Ansprüche 1-11.

## Claims

1. An optical element for efficient generation of short-wave laser light by frequency conversion by means of a wave-guiding layer of a liquid crystalline material having a periodic structure which permits a quasi-phase matching of a guided laser beam, the period length of the three-dimensional structure being defined by the coherence length $l_C = \pi/\Delta\beta$ of the material, where $\Delta\beta = \beta_0(2\omega)-2\beta_0(\omega)$, where $\omega$ = the angular frequency of the fundamental wave, 0 is zero-order mode and $\beta$ = propagation constant of the mode, characterised in that the period length of the three-dimensional structure is equal to twice the coherence length $l_C$
and in that the liquid crystalline material

   contains either a ferroelectric $X^{(2)}$-active mixture of chiral molecules with an nlo molecule group, whose $X^{(2)}$-active axis is directed substantially transversely of the longitudinal axis of the chiral molecule, and SC-phase-forming liquid crystal molecules,

   or an $X^{(2)}$-active, SC*-forming side-chain copolymer, which has chiral side chains with an nlo molecule group, whose $X^{(2)}$-active axis is directed substantially transversely of the longitudinal axis of the side chain.

2. An optical element according to claim 1, characterised in that the material, owing to its birefringent properties, can be used for phase matching of the $d_{21}$ component of the nlo tensor.

3. An optical element according to claim 1 or 2, characterised in that the nlo-molecule group incorporated transversely to the longitudinal axis is a nitroaniline derivative.

4. An optical element according to any of claims 1 to 3, characterised in that the ferroelectric liquid crystal material forms an $S_C$* phase or a higher-order ferroelectric phase which can be dipolar oriented in the electric field.

5. An optical element according to any of claims 1 to 3, characterised in that the ferroelectric LC material forms an $S_C$* phase which is homogeneously oriented in bistable manner by boundary forces.

6. An optical element according to claim 4, characterised in that the dipolar orientation is fixed by cooling below the glass temperature.

7. An optical element according to claim 1 or 2, characterised in that, by using the Pockels' effect, it operates as an electro-optical switch or light modulator in the spectral range from 1300 to 430 nm.

8. An optical element according to any of claims 1 to 3, characterised in that the mixture has the phase sequence I-Chol-$Sm_A$-$S_C$*-glass.

9. An optical element according to claim 1 or 2, characterised in that the element is operated in the $S_C$* phase so that, when an electric field is applied, the $X^{(2)}$ property becomes operative but disappears when the electric field is switched off.

10. An optical element according to claim 1 or 2 characterised in that it is usable for doubling the frequency of laser light having a wavelength of 850 - 1300 nm.

11. An optical element according to any of claims 4 - 6, characterised in that the dipolar orientation is at right angles to the waveguide plane.

12. A frequency-doubling module comprising a laser diode emitting in the range from 850-1300 nm and optically coupled to an optical element according to any of the preceding claims.

13. Compounds having the general formula:

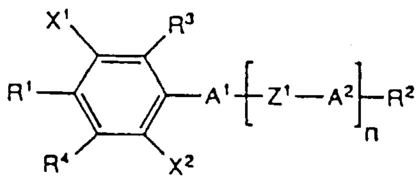

I

where

$X^1$, $X^2$ denote $-NO_2$ and $-NR^5R^6$ respectively, where $R^5$ and $R^6$ independently of one another stand for H or lower alkyl;

$R^3$, $R^4$ independently denote H, F, Cl, Br, $NH_2$, $NO_2$, CN, lower alkyl or lower alkoxy, with the proviso that $X^1$ is different from $R^4$ and $X^2$ is different from $R^3$;

$R^1$ denotes chiral alkyl or cycloalkyl where, independently of one another, one or two non-neighbouring $-CH_2$ groups can be substituted by O, S, -COO-, -OOC-, -HC=CH-, or by -C≡C- in the aliphatic radical, and which can be substituted one or more times by F, Cl, CN, lower alkyl or lower alkoxy, with the proviso that the chirality centre is in an alkyl chain on the first or second C atom, or on a C atom in the ring, in the case of a cycloalkyl radical;

$A^1$ denotes 1 to 4 six-member rings which are directly linked to one another or to the p-nitro-aniline ring and/or at one or optionally a number of places via $-CH_2-CH_2-$, $-CH_2O-$, $-OCH_2-$, -COO-, -OOC-, -COS-, -SOC-, -CH=CH-, -C≡C-, -N=N-, -N=NO-, -ON=N-, -CH=N-, -N=CH-, or butylene, in which a $-CH_2CH_2-$ group can be substituted by -COO-, -OOC-, -HC=CH-, or -C≡C- and/or a $-CH_2$ group can be substituted by O or S. The six-member rings in $A^1$, independently of one another, stand for 1,4-phenylene, unsubstituted or optionally mono-, di- or polysubstituted with F, Cl, lower alkyl or lower alkoxy and in which one or two -CH- groups can be replaced by nitrogen, or 1,4-cylclohexylene in which one or two $-CH_2-$ groups (preferably not neighbouring) can be substituted by O or S or a $-CH_2CH_2-$ group can be subtituted by -CH=CH-, or naphthalene-2,5-diyl, decalin-2,6-diyl, tetralin-2,6-diyl, thiadiazolyl or oxodiazolyl;

Z1 denotes a simple covalent bond, $-CH_2-CH_2-$, $-CH_2O-$, $-OCH_2-$, -COO-, -OOC-, -COS-, -SOC-, -CH=CH-, -C≡C-, -N=N-, -N=NO-, -ON=N-, -CH=N, -N=CH-, or butylene, where a $-CH_2CH_2-$ group can be substituted by -COO-, -OOC-, -HC=CH-, or -C≡C- and/or a $-CH_2-$ group can be substituted by O or S;

$A^1$ denotes the structural element

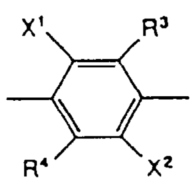

II

where $X^1$, $X^2$, $R^3$ and $R^4$ have the meanings given previously;

n = 0 or 1;

$R^2$ denotes chiral or achiral alkyl or cycloalkyl in which, independently of one another, one or more $-CH_2-$ groups can be substituted by O, S, -COO-, -HC=CH- or by -C≡C- or 1,4 phenylene in the aliphatic radical, and which be substituted one or more times by F, Cl, CN, lower alkyl or lower alkoxy, with the proviso that any chirality centre present will be in an alkyl chain on the first or second C atom or, in the case of a cylcoalkyl radical, will be on a carbon atom in the ring, or if n = O, also the group

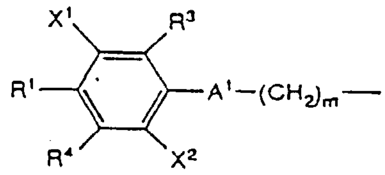

III

where $X^1$, $X^2$, $R^1$. $R^3$, $R^4$ and $A^1$ have the meanings given previously, and M denotes an integer 6-16, where one or two non-neighbouring $-CH_2-$ groups, independently of one another, can be substituted by O, S, -COO-, -CH=CH- or -C≡C-.

**14.** Formula I compounds according to claim 13, characterised in that $R^2$ denotes chiral or achiral alkyl or cycloalkyl in which, independently of one another, one or two non-neighbouring $-CH_2-$ groups can be substituted by O, S, -COO-, -HC=CH- or can be substituted by -C≡C- in the aliphatic radical, and which can be substituted one or more times by F, Cl, CN, lower alkyl or lower alkoxy, with the proviso that any chirality centre present will be in an alkyl chain on the first or second C atom and, in the case of a cylcoalkyl radical, will be on a carbon atom in the ring, or if n = O, also the group

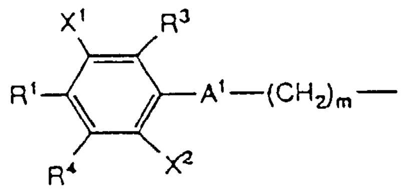

III

where $X^1$, $X^2$, $R^1$. $R^3$, $R^4$ and $A^1$ have the meanings given previously, and m denotes an integer 6-16, where one or two non-neighbouring $-CH_2-$ groups, independently of one another, can be substituted by -O-, -S-, -COO-, -CH=CH- or -C≡C-.

**15.** Non-linear optical compounds according to claim 13 of the general formula Ia

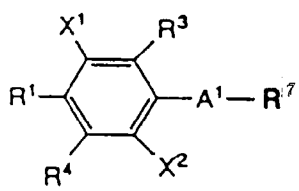

Ia

wherein $R^7$ denotes chiral or achiral alkyl in which, independently of one another, one or more $-CH_2-$groups can be substituted by -O-, -S-, -COO-, -HC=CH-, -C≡C- or 1,4-phenylene, and which can be substituted one or more times by F, Cl, CN, lower alkyl or lower alkoxy.

**16.** Non-linear optical compounds according to claim 13 or 15, characterised in that $R^2$ or $R^7$ bears one of the following polymerisable groups in the terminal position:

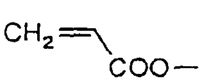 , 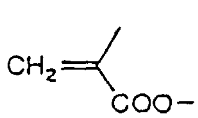 , 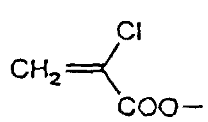 , 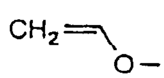 , 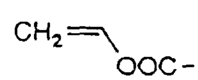

or

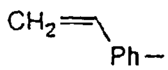

**17.** Use of the non-linear optical compounds according to claim 15 or 16 to form non-linear optical polymers.

**18.** Use of the non-linear optical compounds according to any of the claims 13 to 16 in an optical element according to any of claims 1 to 11.

**19.** Use of the non-linear optical polymers according to claim 17 in an optical element according to any of claims 1 to 11.

**Revendications**

**1.** Dispositif optique pour la production efficace de lumière laser de courte longueur d'onde par conversion de fréquence, comportant une couche guide d'ondes constituée d'un matériau du type cristaux liquides qui présente une structure périodique qui permet un quasi-accord de phase d'un rayon laser guidé, la période de la structure spatiale étant définie par la longueur de cohérence $l_c = \pi/\Delta\beta$ du matériau, où $\Delta\beta = \beta_0 (2\omega) - 2 \beta_0 (\omega)$ avec $\omega$ = pulsation de l'onde fondamentale, 0 = mode d'ordre 0 et $\beta$ = constante de propagation du mode,
caractérisé

par le fait que la période de la structure spatiale correspond au double de la longueur de cohérence $l_c$
et que le matériau du type cristaux liquides contient
soit un mélange ferro-électrique à activité $\chi^{(2)}$ de molécules chirales contenant un groupe de molécules à pouvoir optique non linéaire, dont l'axe d'activité $\chi^{(2)}$ est sensiblement orienté perpendiculairement à l'axe longitudinal de la molécule chirale, ainsi que des molécules de cristaux liquides formant des phases smectiques C,
soit un copolymère à chaîne latérale formant des phases smectiques C chirales $S_c$* à activité $\chi^{(2)}$, qui possède des chaînes latérales chirales contenant un groupe de molécules à pouvoir optique non linéaire, dont l'axe d'activité $\chi^{(2)}$ est sensiblement orienté perpendiculairement à l'axe longitudinal de la chaîne latérale.

**2.** Dispositif optique selon la revendication 1, caractérisé par le fait que, du fait de sa propriété biréfringérente, le matériau permet une adaptation de phase des composants $d_{21}$ du tenseur des coefficients du pouvoir optique non linéaire.

**3.** Dispositif optique selon l'une des revendications 1-2, caractérisé par le fait que le groupe de molécules à pouvoir optique non-linéaire inséré perpendiculairement à l'axe longitudinal, est un dérivé de la nitro-aniline.

**4.** Dispositif optique selon l'une des revendications 1-3, caractérisé par le fait que le matériau ferro-électrique du type à cristaux liquides forme une phase smectique C chirale $S_C$* ou une phase ferro-électrique d'ordre plus élevé qui peut présenter une orientation dipolaire dans le champ électrique.

**5.** Dispositif optique selon l'une des revendications 1-3, caractérisé par le fait que le matériau ferro-électrique du type cristaux liquides forme une phase smectique C chirale qui présente une orientation homogène bistable sous l'action de forces marginales.

**6.** Dispositif optique selon la revendication 4, caractérisé par le fait que l'orientation dipolaire est fixée par refroidissement en dessous de la température de transformation vitreuse.

**7.** Dispositif optique selon l'une des revendications 1-2, caractérisé par le fait qu'il travaille en employant l'effet Pockel comme interrupteur électro-optique ou modulateur de la lumière sur la plage spectrale de 1300 à 430 nm.

**8.** Dispositif optique selon l'une des revendications 1-3, caractérisé par le fait que le mélange présente la succession de phases isotrope-cholestérique-smectique A-smectique chirale C-verre.

**9.** Dispositif optique selon l'une des revendications 1-2, caractérisé par le fait que l'élément qui se trouve dans la

phase smectique chirale C est employé de telle façon que lorsqu'on lui applique un champ électrique, la caractéristique $\chi^{(2)}$ devient active et disparaît lors de la suppression du champ électrique.

**10.** Dispositif optique selon l'une des revendications 1-2, caractérisé par le fait qu'il peut s'employer pour doubler la fréquence de la lumière laser de la longueur d'onde 850-1300 nm.

**11.** Dispositif optique selon l'une des revendications 4-6, caractérisé par le fait que l'orientation dipolaire est perpendiculaire au plan du guide d'ondes.

**12.** Module permettant de doubler la fréquence, constitué d'une diode laser à émission sur la plage de 850-1300 nm optiquement couplée à un dispositif optique selon l'une des revendications précédentes.

**13.** Composés de la formule générale

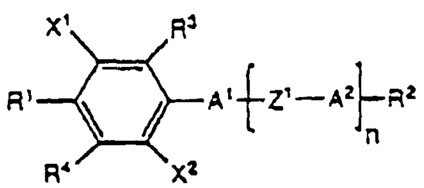

I

avec

$\chi^1$,$\chi^2$: l'un, $-NO_2$ et l'autre, $-NR^5R^6$, $R^5$ et $R^6$ représentant, indépendamment l'un de l'autre, H ou un alkyle d'ordre inférieur;

$R^3$, $R^4$: indépendamment l'un de l'autre, H, F, Cl, Br, $NH_2$, $NO_2$, CN, radical alkyle d'ordre inférieur ou radical alkoxy d'ordre inférieur, avec la condition que $X^1$ soit différent de $R^4$ et $X^2$, différent de $R^3$ ;

$R^1$: radical alkyle ou cycloalkyle chiral dans lequel, indépendamment l'un de l'autre, un ou deux groupes -$CH_2$, non voisins, peuvent être remplacés par O, S, -COO-, -OOC-, HC= CH-, et également, dans le radical aliphatique, par -C≡C-, et qui peut faire, une ou plusieurs fois, l'objet d'une substitution par F, Cl, CN, un alkyle d'ordre inférieur et un alcoxy d'ordre inférieur, avec la condition que le centre de chiralité se trouve, dans une chaîne alkyle, sur le premier ou le second atome C et, dans le cas d'un radical cycloalkyle, sur un atome C du noyau cyclique;

$A^1$: 1 à 4 noyaux cycliques à six éléments, qui sont directement reliés l'un à l'autre ou au noyau cyclique p-nitro-aniline et/ou, en un endroit ou éventuellement plusieurs endroits, sont reliés par l'intermédiaire de $-CH_2-CH_2-$, $-CH_2O-$, $-OCH_2-$, -COO-, OOC-, -COS-, -SOC-, -CH=CH-, -C≡C-, -N=N-, -N=NO-, -ON=N-, -CH=N-, -N=CH-, ou butylène, étant précisé qu'un groupe $-CH_2CH_2-$ peut être remplacé par -COO-, -OOC-, -HC=CH-, -C≡C- et/ou un groupe $-CH_2-$, par O ou S. Les noyaux cycliques à six éléments dans $A^1$ signifient, indépendamment l'un de l'autre, un 1,4-phénylène, n'ayant pas fait l'objet de substitution ou éventuellement ayant fait l'objet d'une mono-, d'une di-, ou d'une poly-substitution par F, Cl, un alkyle d'ordre inférieur ou un alcoxy d'ordre inférieur, et dans lequel 1 ou 2 groupes -CH- peuvent être remplacés par de l'azote, ou bien un 1,4-cyclohexylène, dans lequel un ou deux groupes $-CH_2-$ (de préférence non voisins) peuvent être remplacés par O ou S ou un groupe $-CH_2CH_2-$, par -CH=CH-, ou le naphthalin-2,5-diyle, le décalin-2,6-diyle, le tétraline-2,6-diyle, le thiadiazolyle ou l'oxodiazolyle.;

Z1: une liaison de covalence simple, $-CH_2-CH_2-$, $-CH_2O-$, $-OCH_2-$, -COO-, -OOC-, -COS-, -SOC-, -CH=CH-, -C≡C-, -N=N-, -N=NO-, -ON=N-, -CH=N-, -N=CH-, ou butylène, étant précisé qu'un groupe $-CH_2CH_2-$ peut être remplacé par -COO-, -OOC-, -HC=CH-, -C≡C- et/ou un groupe $-CH_2-$, par O ou S.

$A^2$: L'élément de structure

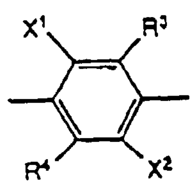

II

où $X^1$, $X^2$, $R^3$ et $R^4$ ont la signification précédemment décrite;

n: le nombre 0 ou 1;

$R^2$: alkyle ou cycloalkyle chirale ou achiral dans lequel, indépendamment l'un de l'autre, un ou deux groupes $-CH_2-$, peuvent être remplacés par O, S, -COO-, -HC= CH-, et également, dans le radical aliphatique, par -C≡C-, ou 1,4 phénylène et qui peut faire, une ou plusieurs fois, l'objet d'une substitution par F, Cl, CN, un alkyle d'ordre inférieur et un alcoxy d'ordre inférieur, avec la condition qu'un centre de chiralité, éventuellement existant, se trouve, dans une chaîne alkyle, au premier ou au second atome C et, dans le cas d'un radical cycloakyle, à un atome C du noyau cyclique, ou, dans le cas où n signifie le nombre 0, également le groupe

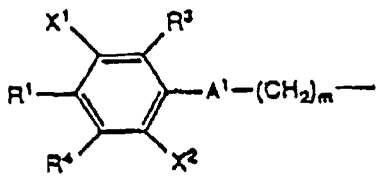

III

où $X^1$, $X^2$, $R^1$· $R^3$, $R^4$ et $A^1$ ont la signification précédente et m indique un nombre entier 6-16, un ou deux groupes $-CH^2-$, non voisins, pouvant, indépendamment l'un de l'autre, être remplacés par O, S, -COO-, -CH=CH- ou -C=C-.

**14.** Composés de la formule I selon la revendication 13, caractérisés par le fait que $R^2$ signifie un alkyle ou un cycloalkyle chiral ou achiral, dans lequel, indépendamment l'un de l'autre, un ou deux groupes $-CH_2-$, peuvent être remplacés par -O-, -S-, -COO-, -HC=CH-, et également, dans le radical aliphatique, par -C≡C-, et qui peut faire, une ou plusieurs fois, l'objet d'une substitution par F, Cl, CN, un alkyle d'ordre inférieur et un alcoxy d'ordre inférieur, avec la condition qu'un centre de chiralité, éventuellement existant, se trouve, dans une chaîne alkyle, au premier ou au second atome C et, dans le cas d'un radical cycloakyle, à un atome C du noyau cyclique, ou, dans le cas où n signifie le nombre 0, également le groupe

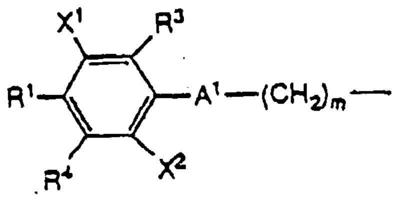

III

où $X^1$, $X^2$, $R^1$, $R^3$, $R^4$ et $A^1$ ont la signification précédente et m indique un nombre entier 6-16, un ou deux groupes $-CH^2-$, non voisins, pouvant, indépendamment l'un de l'autre, être remplacés par O, S, -COO-, -CH=CH- ou -C≡C-.

**15.** Composés à pouvoir optique non linéaire selon la revendication 13 de la formule générale

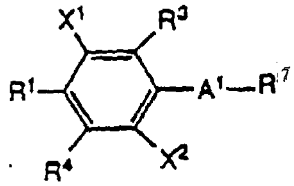

Ia

dans laquelle $R^7$ signifie un alkyle chiral ou achiral dans lequel, indépendamment de l'autre, un ou plusieurs groupes $-CH^2-$ peuvent être remplacés par -O-, -S-, -COO-, -HC-CH- ou -C≡C- ou par 1,4-phénylène et qui peut faire, une fois ou plusieurs fois, l'objet d'une substitution par F, Cl, CN, un alkyle d'ordre inférieur et un alcoxy d'ordre inférieur.

**16.** Composés à pouvoir optique non linéaire selon l'une des revendications 13 ou 15, caractérisés par le fait que $R^2$

ou $R^7$ porte, en position finale, l'un des groupes polymérisables suivants:

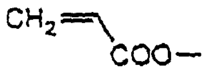, 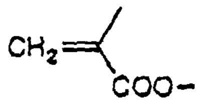, 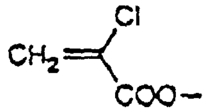, 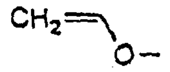, 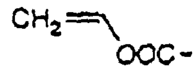

ou

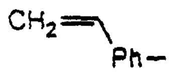

**17.** Emploi des composés à pouvoir optique non linéaire selon l'une des revendications 5 ou 6 pour former des polymères à pouvoir optique non linéaire.

**18.** Emploi des composés à pouvoir optique non linéaire selon l'une des revendications 13 à 16 dans un élément optique conforme à l'une des revendications 1-11.

**19.** Emploi des polymères à pouvoir optique non linéaire selon la revendication 17, dans un élément optique conforme à l'une des revendications 1-11.

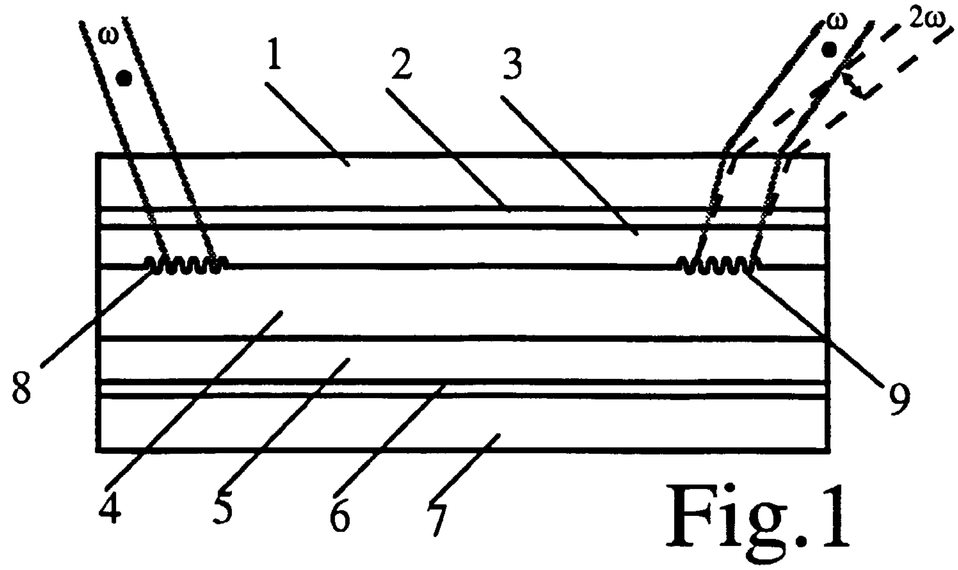


Fig.1

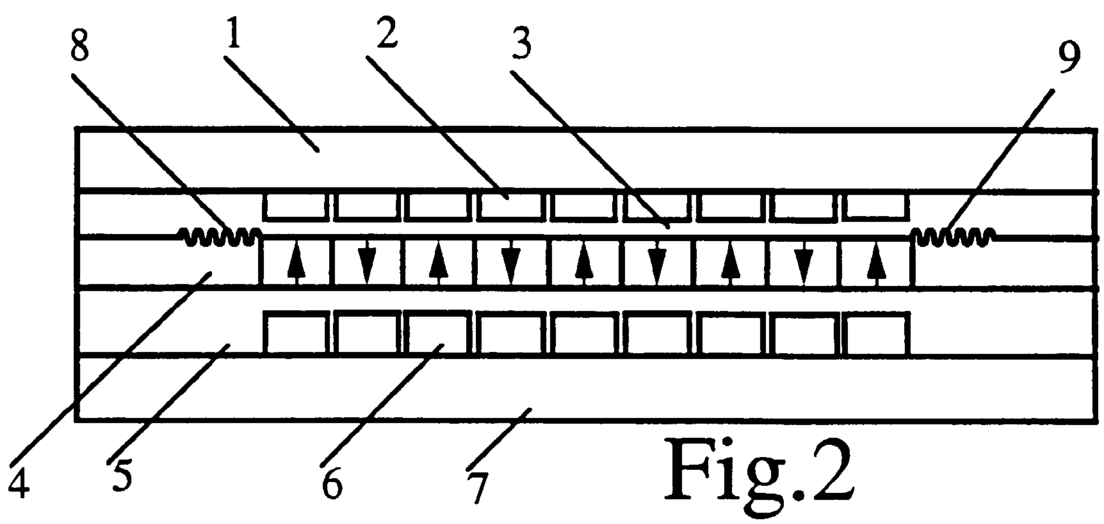


Fig.2